# EUROPEAN PATENT SPECIFICATION

(11) **EP 3 943 935 B1**
(45) Date of publication and mention of the grant of the patent: **07.08.2024**
(21) Application number: 20784727.8
(22) Date of filing: 26.03.2020
(51) Int. Cl.: G01N 35/00, G01N 33/49

(54) **FULLY-AUTOMATIC THROMBOELASTOGRAPHY TESTING DEVICE AND TESTING METHOD**
VOLLAUTOMATISCHE THROMBOZYTENTESTVORRICHTUNG UND TESTVERFAHREN
DISPOSITIF DE TEST DE THROMBOÉLASTOGRAPHIE ENTIÈREMENT AUTOMATIQUE ET PROCÉDÉ DE TEST

(30) Priority: 29.03.2019 CN 201910251281
(43) Date of publication of application: 26.01.2022
(73) Proprietor: Hemoassay Science and Technology (Suzhou) Co., Ltd., Suzhou, Jiangsu 215000 (CN)
(72) Inventor: XIAO, Jian, Suzhou, Jiangsu 215000 (CN); HU, Xi, Suzhou, Jiangsu 215000 (CN); CHEN, Jienian, Suzhou, Jiangsu 215000 (CN); WANG, Tao, Suzhou, Jiangsu 215000 (CN); JIANG, Feng, Suzhou, Jiangsu 215000 (CN)
(74) Representative: Wang, Bo
(86) International application number: PCT/CN2020/081459
(87) International publication number: WO 2020/200046

(56) References cited:
- WO-A1-2014/031253
- WO-A1-2017/121288
- WO-A1-2018/137766
- CN-A- 104 614 539
- CN-A- 106 124 786
- CN-A- 106 370 829
- CN-A- 107 014 990
- CN-A- 107 505 456
- CN-A- 108 562 753
- CN-A- 109 946 444
- CN-U- 210 071 834

## Description

### TECHNICAL FIELD OF THE INVENTION

The invention relates to a thrombo elastometer, in particular to a full-automatic thrombo elastometer device and a test method.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

The physical properties of testing a blood clot are based on the following principles. A special cylindrical cup statically containing blood rotates at a low angular speed of 4.75, and a testing rod immersed in the blood sample is used to detect the movement of the blood sample. After the blood cup adheres to the testing rod through a fibrin platelet complex, the rotational force generated by rotating the cup can be transmitted to the testing rod in the blood sample. The strength of the fibrin platelet complex can influence the movement amplitude of the testing rod, so that a strong blood clot can synchronize the movements of both the testing rod and the blood cup. Therefore, the movement amplitude of the testing rod is directly related to the strength of the formed blood clot. In the case that the blood clot retracts or dissolves, the joint between the testing rod and the blood clot is released, so the movement of the blood cup is no longer transmitted to the testing rod, the rotation of which is detected by the sensor.

As for the current thrombo elastometer, when detecting blood coagulation data, it is necessary to manually take cups, load cups, add blood and reagents to the testing cup, and unload cups, etc., which requires a lot of time for operators, resulting in high labor intensity and low test efficiency for detecting blood coagulation data.

CN 107 014 990 A discloses a full-automatic thrombus elastic force measuring device which comprises a base, a sample component, a sample-feeding driving mechanism, a sample-feeding mixing component, a measuring component and a main control component. CN 107 505 456 A discloses a full-automatic detection type thromboelastography and an application method thereof.

### SUMMARY OF THE INVENTION

An objective of the invention is to overcome the defects of the prior art and provide a full-automatic thrombo elastometer device and a test method.

The invention is set out in the appended set of claims.

The beneficial effects of the present invention are as follows. A testing cup-loading device, a test passage, a reagent-storing device and a blood sample-loading device, etc. are integrated into one housing in the invention by matching two mechanical hands (test-driving device and liquid-adding device) with each other, so as to achieve fully automatically detecting the blood coagulation data, improve test efficiency and test accuracy.

### BRIEF DESCRIPTION OF THE DRAWINGS

FIG 1 is a 3D structure schematic diagram after assembling the outer housing in the invention.
FIGs.2-3 are 3D structure schematic diagrams at two angles after removing the outer housing in the invention.
FIG 4 is a 3D structure schematic diagram of the testing cup-loading device in the invention.
FIG 5 is an exploded view of FIG 4.
FIG 6 is a 3D structure schematic diagram of the testing cup tray from a top view in the invention.
FIG 7 is a structure schematic diagram of the first rotating disk from a top view.
FIG.8 is a 3D structure schematic diagram of the invention (not including the rotationally-driving mechanism).
FIG9 is an overall 3D structure schematic diagram of the invention except the horizontally-driving mechanism.
FIG 10 is a top view of FIG 9.
FIG 11 is a 3D structure schematic diagram of the test passage (without the heat-preserving cover) in the invention.
FIG 12 is an enlarged view of the part circled in FIG 11.
FIG 13 is a 3D structure schematic diagram of the test passage at another angle in the invention.
FIG 14 is a 3D schematic diagram of the reagent-storing device in the invention.
FIG 15 is a cross-sectional view of the reagent-storing device in the invention.
FIG 16 is a 3D schematic diagram of the transmitting base in the invention.
FIG 17 is a 3D schematic diagram of the transmitting drawer in the invention.
FIG 18 is a bottom view of FIG 17.
FIG 19 is a 3D schematic diagram of the refrigerating mechanism in the invention.
FIG20 is a front view of the blood sample-loading device in the invention.
FIG21 is a 3D schematic diagram of the rotating disk in FIG 20.
FIG22 is a 3D schematic diagram of the blood sample tray in FIG20.
FIG23 is a bottom view of the tray body in FIG22
FIG24 is a 3D schematic diagram of the transmission mechanism in FIG20.
FIG25 is a block diagram of the fluid path system according to invention.
FIGs.26-28 are block diagrams of different embodiments of the blood sample fluid path system according to the invention.
FIG.29 is a block diagram of the reagent fluid path system according to the invention.
FIG30 is a 3D structure schematic diagram of the liquid-taking device according to the invention.
FIG31 is an enlarged view of the part circled in FIG30.

Wherein:
100- control device;
200- testing cup-loading device; 201- testing cup tray; 202- first rotating disk; 203- rotating disk-driving device; 204- support frame; 205- loading portions; 206- first matching installation portion; 207- second matching installation portion; 209- first polygonal matching surface; 210-limiting portion; 211- second polygonal matching surface; 212- cup hole; 213- first power source; 214- gear assembly; 215- motor shaft; 216- first driving gear; 217- first driven gear; 218-first sensor;
300- test-driving device; 301- cup-clamping mechanism; 302- first up-down-driving mechanism; 303- rotationally-driving mechanism; 304- horizontally-driving mechanism; 305-clamping assembly-mounting seat; 306- second driving source; 308- second driving source; 309-resetting elastic member; 310- clamping arm; 311- clamping arm; 312- second sensor; 313-mounting frame; 314- third driving source; 315- worm; 316- worm wheel; 317- screw rod; 318-up-down-guiding rod; 319- mounting plate; 320- fourth driving source; 321- rotating shaft; 322-fifth driving source; 323- driving screw rod; 324- slider;
400- test passage; 401- ninth driving source; 402- driving rod; 403- beating plate; 404-through-hole; 405- testing rod; 406- guiding rod; 407- cup lid; 408- heat-preserving cover; 409-rotating mechanism; 410- second driving source; 411- cam; 412- swinging arm; 413-up-down-driving mechanism; 414- motor; 415- screw rod; 416- cup holder; 417- sliding stand; 418- support;
500- reagent-storing device; 501- housing; 502- transmitting base; 503- transmitting drawer; 504- refrigerating mechanism; 505- locking assembly; 506- rotating member; 507- first elastic member; 508- tracking groove; 509- locking pin; 510- opening; 511- first guiding groove; 512-limiting groove; 513- second guiding groove; 514- placing groove; 515- second elastic member; 516- guiding member; 517- containing groove; 518- guiding rail; 519- sliding groove; 520-drawer body; 521- transmitting carrier; 522- drawer panel; 523- refrigerating member; 524- heat dissipating member; 525- fan; 526- air inlet; 527- installation plate; 528- liquid-taking opening;
600- blood sample-loading device; 601- second rotating disk; 602- blood sample tray; 603-regular polygonal convex portion; 604- regular polygonal concave portion; 605- tray body; 606-carrier; 607- second opening; 608- fixing seat; 609- blood sample testing tube-loading seat; 610-transmission mechanism; 611- transmission shaft; 612- second driving wheel; 613- second driven wheel; 614- motor; 615- barcode reader; 616- plane light; 617- containing groove;
700- outer housing;
800- testing cup;
900- fluid path system; 901- blood sample fluid path system; 902- reagent fluid path system; 903- blood-suctioning member; 904- first suction-controlling member; 905- suctioning member-cleaning device; 906- first control valve; 907- air source; 908- water source; 909- check valve; 910- first diaphragm pump; 911- suctioning-member outer-wall-cleaning device; 912-suctioning member-cleaning trough; 913- second diaphragm pump; 914- sewage-collecting device; 915- third diaphragm pump; 916- sewage barrel; 917- sewage outlet; 918-reagent-suctioning member; 919- second suction-controlling member; 920- integrated rod; 921-connection opening; 922- water source inlet; 923- connector;
930- liquid-taking device; 931- blood suction-driving mechanism; 932- reagent suction-driving mechanism; 933- rotationally-driving mechanism; 934- first screw rod; 935-first sliding stand; 936- first mounting plate; 937- first gear; 938- second gear; 939- second mounting plate; 940- rotary bearing; 941- first sliding rail; 942- first fixing bump; 943- seventh driving source; 944- second screw rod; 945- second sliding stand; 946- second sliding rail; 947-second fixing bump; 948- base; 949- eighth driving source; 950- rotating shaft; 951- third motor; 952- liquid level sensor; 953- waste-collecting device; 954- tenth driving source.

### DETAILED DESCRIPTION OF THE INVENTION

The technical solutions of the embodiments in the invention will be clearly and completely described below in combination with the figures in the invention.

A full-automatic thrombo elastometer device and a test method disclosed by the invention achieve fully automatically taking cups, loading cups, adding blood and reagents to the testing cup, testing, and unloading cups after testing, etc., during detecting blood coagulation data.

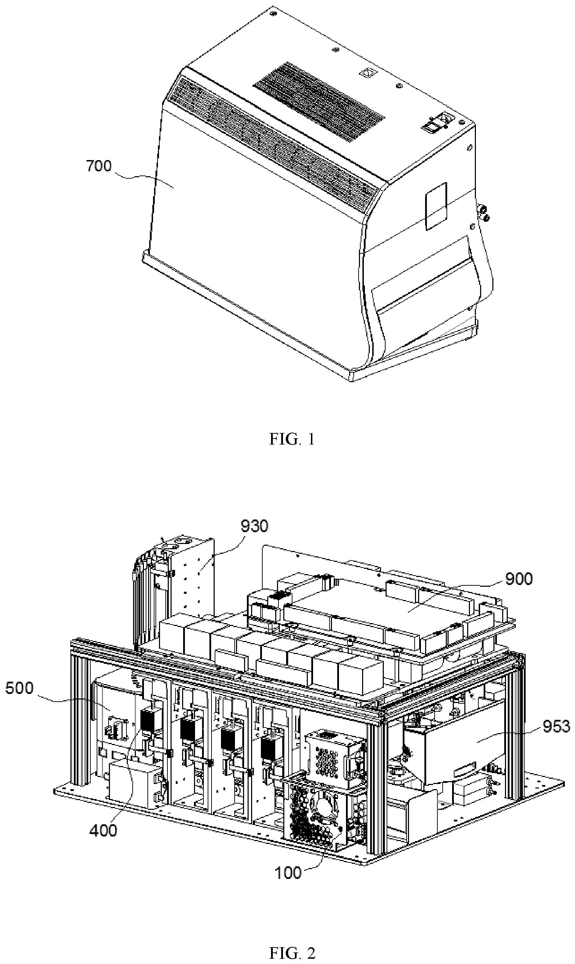

As shown in FIGs.1-3, a full-automatic thrombo elastometer device disclosed by the invention includes a control device 100, a testing cup-loading device 200 connected to the control device 100, a test-driving device 300, a plurality of test passages 400, a reagent-storing device 500, a blood sample-loading device 600, and a liquid-adding device, in this embodiment, these devices are all integrated in an outer housing 700. The test-driving device 300 inside the outer housing 700 matches with the liquid-adding device, completely replaces manual labor, and completes fully automatically detecting blood coagulation data.

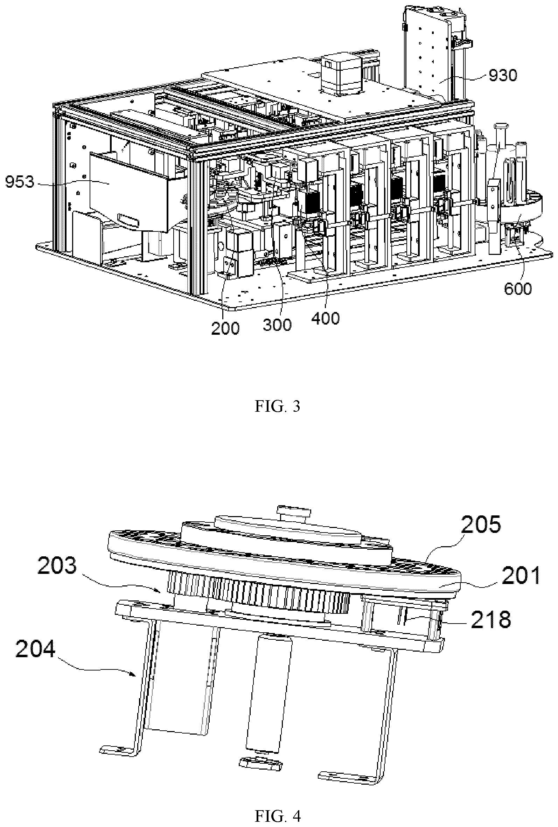

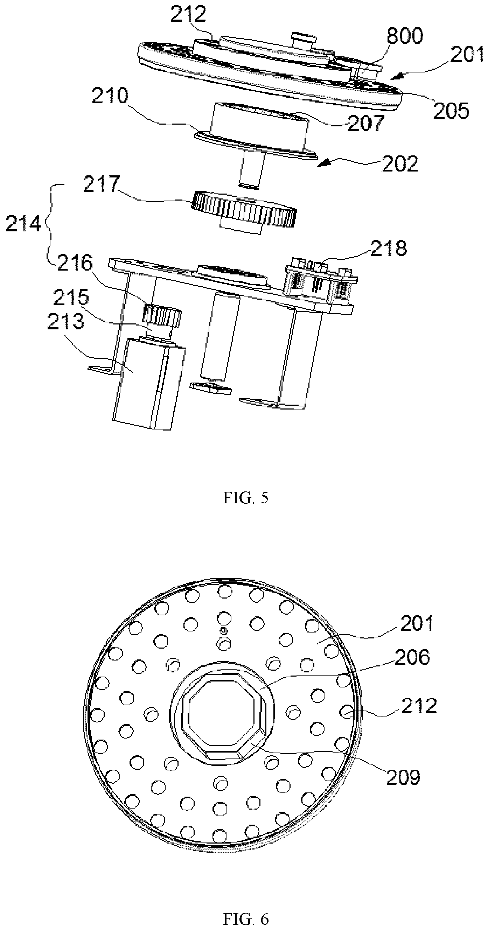

Specifically, as shown in FIGs.4-7, the testing cup-loading device 200 is used to load the testing cup required during a blood coagulation test. It specifically includes a testing cup tray 201, a first rotating disk 202, a rotating disk-driving device 203, and a support frame 204, wherein the testing cup tray 201 matches with the first rotating disk 202 by using the polygonal matching surface with different edge numbers arranged in a multiple proportion so as to achieve quickly installing the testing cup tray 201 on the first rotating disk 202.

Specifically, the testing cup tray 201 is integrally formed, is disc-shaped in whole. It specifically includes a plurality of loading portions 205 sequentially arranged in stratification from top to bottom, and the outer diameters of these loading portions 205 increase in the direction extending from the inner side to the outer side of the testing cup tray 201, that is, the outer diameter of the innermost and uppermost loading portion 205 is the smallest, and the outer diameter of the outermost and lower loading portion 205 is the largest, in this way, a step is formed between two adjacent loading portions 205. The stratified structure of the testing cup tray 201 allows the tray to load a larger number of testing cups 800 as required.

Each loading portion 205 is provided with at least one circle of cup holes 212 evenly distributed in the circumferential direction, the cup hole is used for loading the testing cup, and its shape is set in accordance with the shape of the testing cup 800. In this embodiment, 48 testing cups 800 can be loaded in total on the entire testing cup tray 201.

The testing cup tray 201 is installed on the first rotating disk 202, and the testing cup tray 201 is fitted to the first rotating disk 202 through the first matching installation portion 206 and the second matching installation portion 207 matching with each other to achieve quickly install the testing cup tray on the first rotating disk 202. Specifically, the first matching installation portion 206 is formed on the lower surface of the testing cup tray 201, and particularly located at the center of the lower surface, and a concave is formed upwards from the lower surface of the testing cup tray 201, that is, a groove. In addition, the inner surface of the groove is formed as a first polygonal matching surface 209. In this embodiment, the first polygonal matching surface 209 is a regular polygon, specifically a regular octagon.

The first rotating disk 202 specifically includes the above-mentioned second matching installation portion 207 and a limiting portion 210. The second matching installation portion 207 is formed by protruding upwards from the upper surface of the limiting portion 210, and inserted into the first matching installation portion 206. After insertion, the outer surface of the second matching installation portion 207 is formed as a second polygonal matching surface 211, which matches with the first polygonal matching surface 209 of the first matching installation portion 206, and the second polygonal matching surface 211 is also a polygon, but the ratio of its edge number to that of the first polygonal matching surface 209 is 1:n, where n is an integer greater than or equal to 2, that is, the edge numbers of the two matching surfaces are different ( not proportion 1: 1) and a multiple proportion. In this embodiment, the second polygonal matching surface 211 is a 24 regular polygon, that is, the ratio of the edge number of the first polygonal matching surface 209 to that of the second polygonal matching surface 211 is 1:4.

Of course, the above-mentioned first matching installation portion 206 and second matching installation portion 207 are not limited to the above-mentioned structure of the groove and protrusion matching with each other, and can also be replaced by the first matching installation portion 206 having a protruding structure, and the second matching installation portion 207 being a groove structure, as long as both can match with each other by polygonal matching surfaces with different edge numbers arranged in a multiple proportion.

The rotating disk-driving device 203 is connected to the first rotating disk 202, used to drive the first rotating disk 202 to rotate in the radial direction, which helps to match with the test-driving device on the thrombo elastometer to sequentially clamp the testing cup 800. In this embodiment, the rotating disk-driving device 203 specifically includes a first power source 213, a gear assembly 214, and a driving shaft, wherein the first power source 213 may be a motor, and the gear assembly 214 is connected to the motor shaft 215 of motor 213, and connected with the first rotating disk 202 to drive the first rotating disk 202 to rotate in the radial direction. In this embodiment, the gear assembly 214 specifically includes a first driving gear 216 and a first driven gear 217, and the first driving gear 216 is fixedly installed on the motor shaft 215, the first driven wheel 217 meshes with the first driving gear 216, and rotates about the motor shaft 215. The motor shaft 215 is connected to both the first driven wheel 217 and the first rotating disk 202, and driven by the gear assembly 214 to drive the first rotating disk 202 to rotate in the radial direction.

The support frame 204 is used to support and install the testing cup tray 201, the first rotating disk 202 and the rotating disk-driving device 203 all mentioned above.

Preferably, the testing cup-loading device of the invention further includes at least one sensor 218 installed on the support frame 204. The first sensor 218 is arranged close to the testing cup tray 201 for testing whether there is a testing cup on the testing cup tray 201. In this embodiment, three first sensors 218 are installed on the support frame 204, located below the testing cup tray 201. The three first sensors 218 correspond to the three circles of the testing cups on the testing cup tray 201, that is, each circle of the testing cups 800 is correspondingly provided with one first sensor 218.

The working principle of the testing cup-loading device is as follows. The testing cup tray 201 is loaded with the testing cup 800 manually or automatically, and the testing cup tray 201 loaded with the testing cup 800 is quickly installed on the first rotating disk 202, which is driven by the rotating disk-driving device 203 to rotate, so as to convey the testing cup 800 to the position to be clamped by the test-driving device (mechanical hand). While the first rotating disk 202 is rotating, the first sensor 218 senses whether there is a testing cup 800 in the corresponding cup hole 212 on the rotating disk. If it senses that there is no testing cup 800 on the testing cup tray 201, the testing cup tray 201 is removed from the first rotating disk 202, and the device repeats the above process after reloading the testing cup 800.

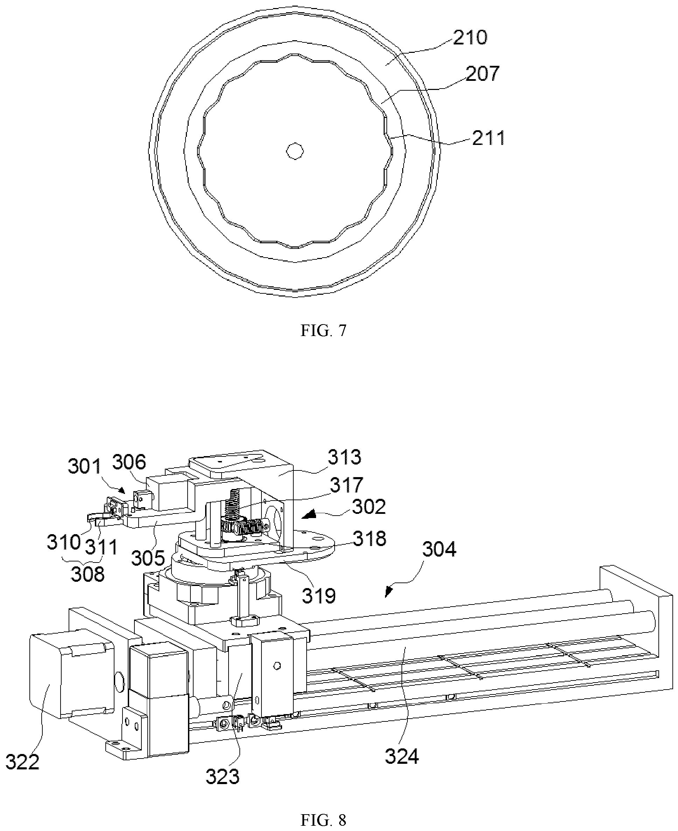

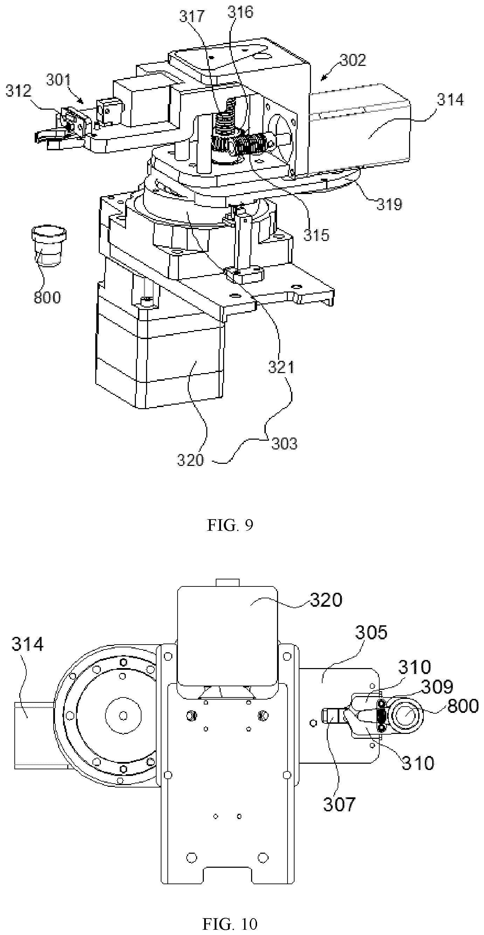

As shown in FIGs.8-10, the test-driving device 300 is used to automatically take cups, load cups, and unload cups during detecting blood coagulation data. In this embodiment, the test-driving device specifically includes a cup-clamping mechanism 301, a first up-down-driving mechanism 302, a rotationally-driving mechanism 303, and a horizontally-driving mechanism 304, The up-down, rotationally and horizontally driving mechanisms 302, 303, and 304 cooperatively drive the cup-clamping mechanism 301 to clamp and unload the testing cup 800 to the testing cup-loading device 200, the test passage 400 and the liquid-adding device.

The cup-clamping mechanism 301 is used to clamp and unload the testing cup 800, in this embodiment, it specifically includes a clamping assembly-mounting seat 305, a second driving source 306, a clamping jaw assembly 308, and a resetting elastic member 309, wherein the second driving sources 306 is fixed on the clamping assembly-mounting seat 305 and moves back and forth in the horizontal direction. when implemented, it can be an electromagnet, the clamping jaw assembly 308 is installed on the clamping assembly-mounting seat 305, and clamps or unloads the testing cup 800, driven by the second driving source 306. In this embodiment, the clamping jaw assembly 308 includes two clamping arms 310 and 311, one end of the two clamping arms 310 and 311 crosses with each other, and the other end forms a clamping jaw with each other. The second driving source 306 is located close to one end of the clamping jaw assembly 308 crossing with each other, moves towards the direction close to the clamping jaw assembly 308 to abut on one end of the clamping jaw assembly 308 crossing with each other, then drives the other end of the clamping jaw assembly 308 to open. The resetting elastic member 309 is arranged between the two clamping arms 310 and 311 at the other end of the clamping jaw assembly 308, used to produce a resetting elastic force that makes the other end of the two clamping arms 310 and 311 retract back, when implemented, the resetting elastic member 309 can be a spring.

Preferably, the cup-clamping mechanism 301 further includes a second sensor 312 installed on the clamping assembly-mounting seat 305 and arranged close to the clamping jaw assembly 308, used for testing the state of the clamping jaw assembly 308 clamping the testing cup 800, such as whether the testing cup 800 is clamped or not.

The first up-down driving mechanism 302 is connected to the cup-clamping mechanism 301 and is used to drive the cup-clamping mechanism 301 to move up and down in a vertical direction. The first up-down-driving mechanism 302 specifically includes a mounting frame 313, an eighth driving source 949, a driving steering assembly and a screw rod 317, wherein the eighth driving source 949 is fixedly installed on the mounting frame 313 and connected with the driving steering assembly, when implemented, the eighth driving source 949 can also be a motor, the driving steering assembly is used to convert the axial rotation of the driving shaft of the eighth driving source 949 into radial rotation, in this embodiment, the driving steering assembly includes a worm 315 connected to the driving shaft of the eighth driving source 949 and a worm wheel 316 matching and meshing with the worm 315, and the worm wheel 316 encircles the screw rod 317 and is threadedly connected with the screw 317, the screw rod 317 is vertically installed on the mounting frame 313 and driven to rotate in the horizontal direction by the worm wheel 316, the screw rod 317 is connected, for example, threadedly connected to the clamping assembly-mounting seat 305 of the clamping-cup mechanism 301, while the screw rod 317 is rotating, the clamping assembly-mounting seat 305 is driven to move up and down in the vertical direction, thereby synchronously driving the clamping jaw assembly 308 to move up and down in the vertical direction, so as to adjust the testing cup 800 on the clamping jaw assembly 308 to a desired height.

Preferably, the up-down-driving mechanism 302 further includes at least one up-down-guiding rod 318 vertically fixed on the mounting frame 313 for orientation, and the cup-clamping mechanism 301 is connected to the up-down-guiding rod 318. Specifically, the clamping assembly-mounting seat 305 of the clamping-cup mechanism 301 passes through the up-down-guiding rod 318, and is driven by the screw rod 317 to move up and down in the vertical direction along the up-down-guiding rod 318.

In addition, in this embodiment, the up-down-driving mechanism 302 and the rotationally-driving mechanism 303 are integrated and mounted on the same mounting plate 319, wherein the up-down-driving mechanism 302 is fixed above the mounting plate 319, and the rotationally-driving mechanism 303 is fixed below the mounting plate 319.

The rotationally-driving mechanism 303 is connected to the cup-clamping mechanism 301, specifically connected to the mounting plate 319, used to drive the entire up-down-driving mechanism 302 to rotate in a second direction (the second direction is the horizontal direction) perpendicular to the above first direction through the mounting plate 319, further synchronously drive the cup-clamping mechanism 301 connected with the up-down-driving mechanism 302 to rotate in the horizontal direction, so that the cup-clamping mechanism 301 rotates to a desired position.

In this embodiment, the rotationally-driving mechanism 303 specifically includes an eighth driving source 949 and a rotating shaft 950 connected to the eighth driving source 949, when implemented, the eighth driving source 949 can also be a motor, the rotating shaft 950 is fixedly connected with the up-down-driving mechanism 302, specifically fixed connected with the above-mentioned mounting plate 319, and drives the up-down-driving mechanism 302 fixed on the mounting plate 319 by driving the mounting plate 319 to rotate in the horizontal direction, and then the up-down-driving mechanism 302 synchronously drives the cup-clamping mechanism 301 connected to it to rotate in the horizontal direction.

The horizontally-driving mechanism 304 is connected to the cup-clamping mechanism 301, specifically fixedly connected to the mounting plate 319, used to drive the cup-clamping mechanism 301 to move horizontally in the above second direction perpendicular (that is the horizontal direction). Specifically, in this embodiment, the horizontally-driving mechanism 304 includes a fifth driving source 322, a driving screw rod 323 and a slider 324,wherein, when implemented, the fifth driving source 322 can also be a motor, the driving screw rod 323 is connected to the fourth driving source 322, the slider 324 is slidably mounted on the driving screw rod 323, and the upper end of the slider 324 is fixedly connected to the mounting plate 319, the driving screw rod 323 drives the slider 324 to move horizontally in the horizontal direction, driven by the fifth driving source 322, the slider 324 drives the rotationally-driving mechanism 303, the up-down-driving mechanism 302 and the cup-clamping mechanism 301 all integrated on the mounting plate 319 to slide in the horizontal direction, so as to make the cup-clamping mechanism 301 move to the corresponding station in the horizontal direction.

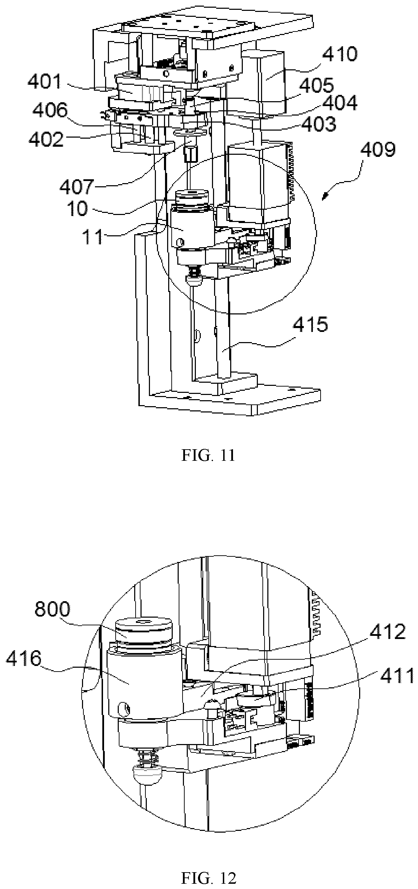

As shown in FIGs.11-13, the test passage 400 is used to achieve detecting blood coagulation data, in this embodiment, 8 test passages 400 are arranged, and located on both sides of the test-driving device 300, each side of which is provided with 4 test passages 400. Each test passage 400 specifically includes a cup lid-beating device, a rotating mechanism 409, and a up-down-driving mechanism 413, wherein the cup lid-beating device includes a ninth driving source 401, a driving rod 402, and a beating plate 403, when implemented, the ninth driving source 401 can be a motor, the driving rod 402 is vertically arranged, one end (here, the upper end) is connected to the ninth driving source 401, and the other end is a free end, which rotates, driven by the ninth driving source 401, in this embodiment, the driving rod 402 is a screw rod, that is, the outer surface is provided with an external thread.

The beating plate 403 traps the driving rod 402, and it is arranged horizontally, that is, perpendicular to the driving rod 402. The inner surface of the beating plate 403 touching the driving rod 402 are provided with internal threads (not shown in the figures), so both are threadedly connected with each other, in this way, when the driving rod 402 is driven by the ninth driving source 401 to rotate, the beating plate 403 can move up and down with respect to the driving rod 402. In this embodiment, the beating plate 403 has a plate structure as a whole, and its other end away from the driving rod 402 traps the testing rod 405, and has a through-hole 404 formed for the testing rod 405 to pass through, but the testing rod 405 does not touch the through-hole 404.

Preferably, in order to guide the beating plate 403 to move up and down, the cup lid-beating device of this embodiment further includes at least one guiding rod 406 vertically arranged, and the beating plate 403 also traps the guiding rod 406, and moves up and down along the guiding rod 406, driven by the driving rod 402.

The other end of the beating plate 403 is located above the cup lid 407 on the testing rod 405, and its plane is parallel or approximately parallel to the plane of the cup lid 407. In addition, in order to further improve the stability of the beating plate 403 and the cup lid 407, the bottom surface area of the other end of the beating plate 403 touching the cup lid 407 is set to be greater than or equal to the area of the upper surface of the cup lid 407, so that the beating plate 403 can completely cover the cup lid 407 when it touches the cup lid 407. In this way, the driving rod 402 is driven by the ninth driving source 401 to drive the beating plate 403 to move in a direction close to the cup lid 407, so that the beating plate 403 touches the cup lid 407 to unload the cup lid 407 from the testing rod 405, compared with the previous lever-driven structure, the cup-beating action is more stable.

More preferably, the testing rod 405 is also provided with a heat-preserving cover 408 that wraps the upper end of the cup lid 407. The heat-preserving cover 408 surrounds the testing rod 405, not touching the testing rod 405, and is connected to the beating plate 403, used to guide the beating plate 403 to move up and down in a vertical direction, while increasing the contact area with the cup lid 407.

The rotating mechanism 409 is used to control the rotation of the testing cup 800 during a test. In this embodiment, the rotating mechanism 409 specifically includes a second driving source 410, a cam 411, and a swinging arm 412. When implemented, the second drive source 410 can also be a motor. The cam 411 is connected to the second driving source 410, and rotates driven by the second driving source 410. One end of the swinging arm 412 touches the cam 411, and the other end is connected to a cup holder 416 for holding the testing cup 800. During operation, the swinging arm 412 drives the cup holder 416 connected to itself to swing synchronously while the cam 411 is rotating, so that the curve characteristics of the cam 411 are used to accurately control the swing amplitude of the cup holder 416.

The up-down-driving mechanism 413 is connected to the cup holder 416, and is used to drive the testing cup 800 in the cup holder 416 to move to a set test height, when implemented, it can include a motor 414, a screw rod 415, a sliding stand 417, and a support 418, wherein the motor 414 is connected to the screw rod 415, the sliding stand 417 is arranged on the screw rod 415, and the rotationally-driving mechanism 8 is connected to the sliding stand 417, the screw rod 415 drives the sliding stand 417 to move up and down, driven by the motor 414, thereby driving the testing cup 800 to move up and down, which will not be repeated here. In addition, the principle and structure of the test passage 400 detecting blood coagulation data can refer to the prior art, and will not be repeated here.

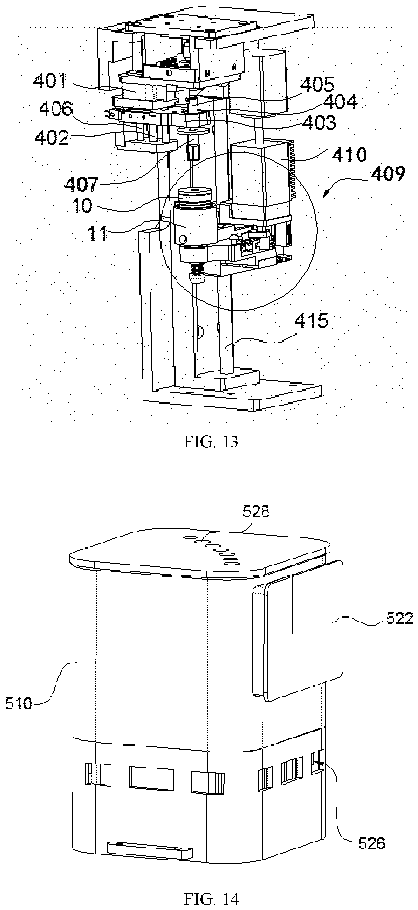

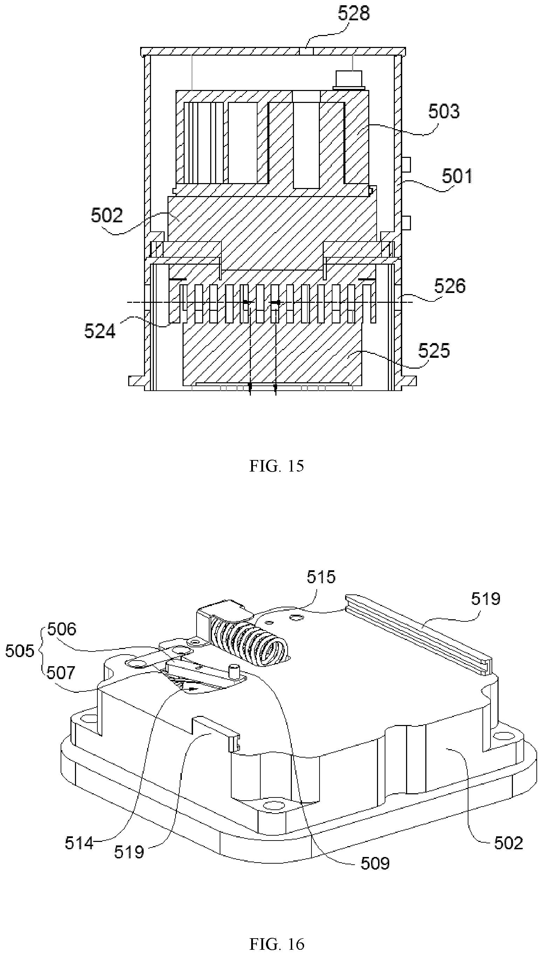

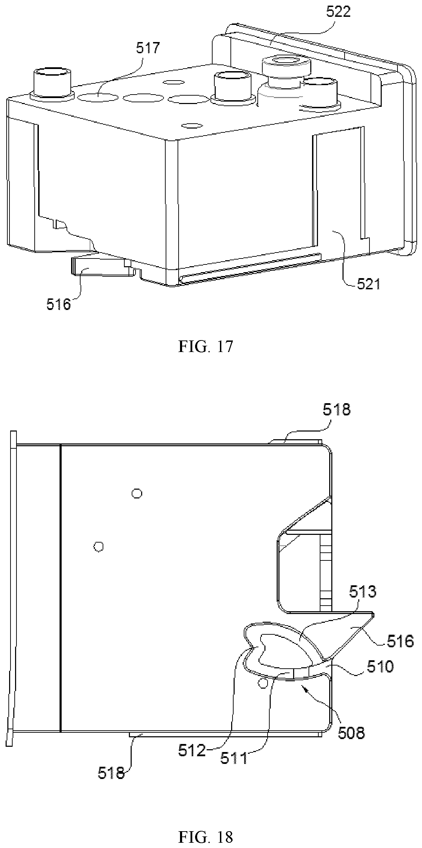

As shown in FIGs.14-19, the reagent-storing device 500 is used for refrigerating reagents and/or blood, in this embodiment, the reagents are refrigerated and to be taken to test for the liquid-adding device. It specifically includes a housing 501, a transmitting base 502, a transmitting drawer 503, an interlocking mechanism, and a refrigerating mechanism 504, wherein the transmitting base 502 is installed in the housing 501, used for heat transfer, the transmitting drawer 503 is located in the housing 501, and is movably installed on the transmitting base 502, used for loading reagents, the refrigerating mechanism 504 is installed in the housing 501, used to generate a cold source, the interlocking mechanism is installed in the housing 501, used to realize the press-locking or press-unlocking between the transmitting drawer 503 and the transmitting base 502.

In specific implementation, the transmitting drawer 503 and the transmitting base 502 can be unlocked by pressing the transmitting drawer 503 under the action of the interlocking mechanism, so as to withdraw the transmitting drawer 503 to place the reagent to be refrigerated in the transmitting drawer 503. After the reagent is placed, the transmitting drawer 503 is fitted on the transmitting base 502 and the transmitting drawer 503 is locked with the transmitting base 502 by pressing the transmitting drawer 503 under the action of the interlocking mechanism. The refrigerating mechanism 504 exchanges heat with the transmitting drawer 503 through the transmitting base 502, and further cools the reagent in the transmitting drawer 503.

The interlocking mechanism includes a locking assembly 505 installed on the transmitting base 502 and a tracking groove 508 installed on the transmitting drawer 503, the press-locking or press-unlocking can be realized between the transmitting drawer 503 and the transmitting base 502 by matching the locking assembly 505 with the tracking groove 508.

Specifically, the locking assembly 505 includes a rotating member 506 and a first elastic member 507, wherein one end of the rotating member 506 is pivotally connected to the transmitting base 502 through a rotating shaft, and the opposite end is provided with a locking pin 509 extending into the tracking groove 508. Under the action of external force, the rotating member 506 can drive the locking pin 509 to rotate around the rotating shaft, and the first elastic member 507 is connected with the rotating member 506 to drive the rotating member 506 to reset.

In this embodiment, the first elastic member 507 is preferably a torsional spring.

The tracking groove 508 includes a first opening 510, a first guiding groove 511, a limiting groove 512 and a second guiding groove 513, wherein, one end of the limiting groove 512 leads to one end of the first guiding groove 511, the opposite end leads to one end of the second guiding groove 513, and the other ends of the first guiding groove 511 and the second guiding groove 513 both lead to the first opening 510. In this embodiment, it is best that the depth of the first guiding groove 511 is greater than that of the second guiding groove 513, so that the locking pin 509 can first enter the first guiding groove 511 when passing through the first opening 510.

In specific implementation, pressing the transmitting drawer 503 can make the transmitting drawer 503 slide on the transmitting base 502, the locking pin 509 enters the first guiding groove 511 passing through the first opening 510, and enters the limiting groove 512 under the action of the first guiding groove 511, during moving the transmitting drawer 503, the transmitting drawer 503 is locked with the transmitting base 502 by matching the limiting groove with 512 with the locking pin 509. Further, continuing to press the transmitting drawer 503 makes the locking pin 509 move to the second guiding groove 513 from the limiting groove 512 under the action of the first elastic member 507, the locking pin 509 finally moves out through the first opening 510, under the action of the second guiding groove 513, the transmitting drawer 503 and the transmitting base 502 are unlocked.

In this embodiment, the limiting groove 512 has a V or U shape as a whole, and the tracking groove 508 has a heart shape as a whole.

The transmitting base 502 is also provided with a placing groove 514, and the locking assembly 505 is installed in the placing groove 514, of course, a placing groove 514 can also be provided on the transmitting drawer 503 and the tracking groove 508 is arranged in the placing groove 514. In this embodiment, it is best to install a placing groove 514 on the transmitting base 502 for assembling the rotating components.

Furthermore, the transmitting base 502 is also provided with a second elastic member 515 used for quickly ejecting the transmitting drawer 503, when implemented, in order to lock the transmitting base 502 with the transmitting drawer 503, the latter slides on the former to make the second elastic member 515 continuously compressed, when the transmitting drawer 503 is locked with the transmitting base 502, the transmitting drawer 503 stops compressing the second elastic member 515. In order to unlock transmitting base 502 and the transmitting drawer 503, pressing the latter to further compress the second elastic member 515, while the locking pin 509 moves from the limiting groove 512 to the second guiding groove 513, a restoring force is generated in the second elastic member 515 to eject the transmitting drawer 503, which facilitates to draw out the transmitting drawer 503 from the housing 501.

The transmitting drawer 503 is also provided with a guiding member 516 that allows the locking pin 509 to quickly enter the tracking groove 508, in this embodiment, the guiding member 516 is triangular as a whole.

Further, the transmitting drawer 503 is provided with a plurality of containing grooves 517 for placing reagents. In this embodiment, the containing grooves 517 are preferably arranged in an arc shape, of course, they can also be arranged according to actual needs.

Furthermore, the transmitting drawer 503 is provided with guiding rails 518 on both sides, and the transmitting base 502 is provided with a sliding groove 519 matching with the guiding rail 518, so the transmitting drawer 503 can be quickly assembled on the transmitting base 502, by matching guiding rail 518 with the sliding groove 519, which improves the assembly accuracy.

In this embodiment, the transmitting drawer 503 adopts a split-type structure, which includes a drawer body 520 and a transmitting carrier 521 installed on the drawer body 520. The transmitting carrier 521 and the transmitting base 502 touch each other, and heat exchange happens between both. Further, a plurality of the containing grooves 517 are provided on the transmitting carrier 521, when implemented, reagents are placed in the containing groove 517 to effect heat exchange with the transmitting carrier 521.

Further, the device also includes a drawer panel 522 connected to the transmitting drawer 503, and the transmitting drawer panel is used to pull out or push in the transmitting drawer 503.

The refrigerating mechanism 504 includes a refrigerating member 523, a heat dissipating member 524, and a fan 525, wherein refrigerating member 523 is installed between the transmitting base 502 and the heat dissipating member 524, and the heat dissipating member 524 is installed between the refrigerating member 523 and the fan 525. Further, the housing 501 is provided with a plurality of air inlets 526 at a position close to the heat dissipating member 524, the cooling air enters the housing 501 through the air inlet 526 and flows through the heat dissipating member 524 to take away the heat on heat dissipating member 524, under the action of the fan 525.

Further, the refrigerating mechanism further includes an installation plate 527 for installing the refrigerating member 523.

When implemented, the refrigerating member 523 generates a cold source when it is energized, and the heat exchange with the reagent performed on the transmitting base 502 and the transmitting carrier 521 so as to bring heat to the heat dissipating member 524. The fan 525 draws cooling air through the air inlet 526, and cooling air takes away the heat on the heat dissipating member 524.

The end of the housing 501 is also provided with several liquid-taking openings 528 matching with reagent bottles, when implemented, a liquid-taking needle passes through the liquid-taking opening 528 and enters the reagents to absorb a reagent.

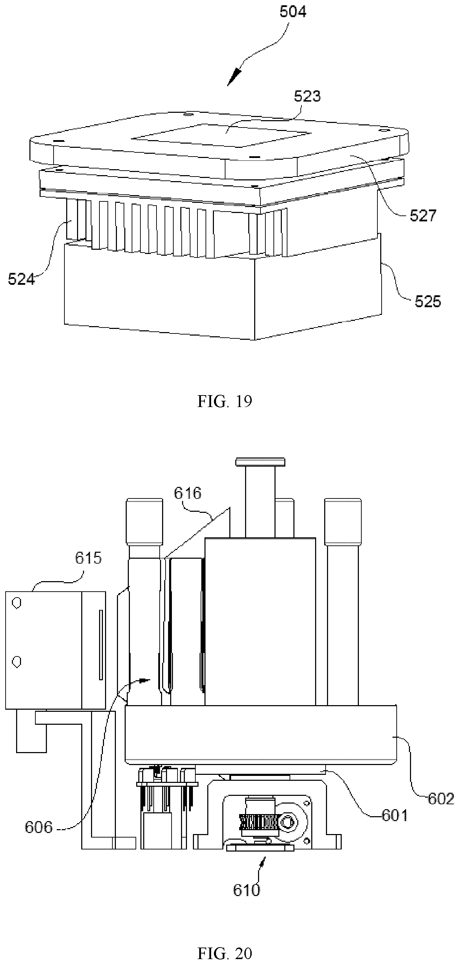

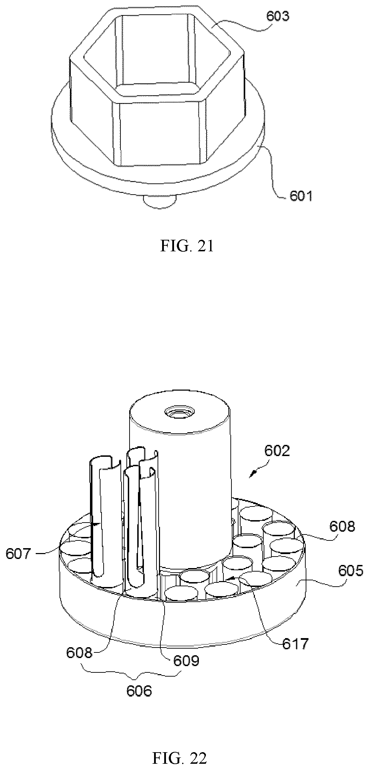

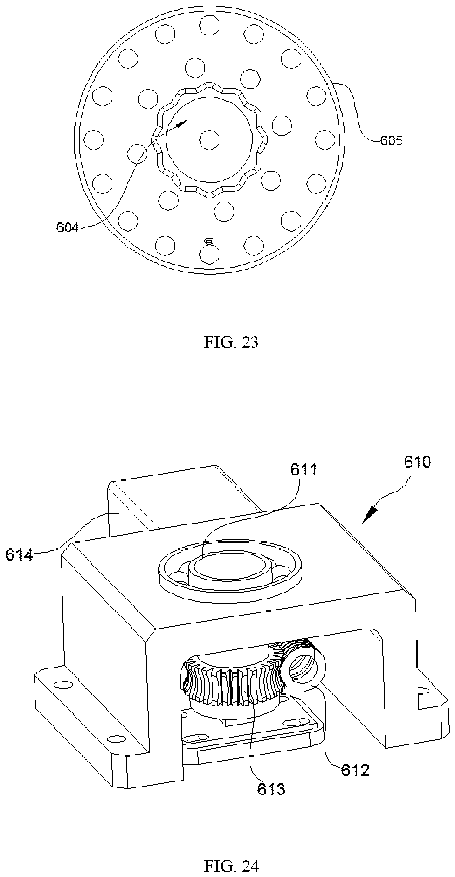

As shown in FIGs.20-24, the blood sample-loading device 600 is used to load blood, and for the liquid-adding device to take a blood sample for testing. It specifically includes a second rotating disk 601, and a blood sample tray 602 installed on the second rotating disk 601 and used to carry blood sample testing tubes containing blood samples. In order to quickly install the blood sample tray 602 on the second rotating disk 601, the second rotating disk 601 is provided with a regular polygonal convex portion 603, and the blood sample tray 602 is provided with a regular polygonal concave portion 604 matching the regular polygonal convex portion 603, the ratio of the edge number of the regular polygonal convex portion 603 to that of the regular polygonal concave portion 604 is at least 1:4; or the second rotating disk 601 is provided with a regular polygonal concave portion 604, and the blood sample tray 602 is provided with a regular polygonal convex portion 603 that matches with the regular polygonal concave portion 604, the ratio of the edge number of the regular polygonal convex portion 603 to that of the regular polygonal concave portion 604 is at least 1:4. The blood sample tray 602 can be quickly assembled to the second rotating disk 601 by setting the regular polygonal convex portion 603 and the regular polygonal concave portion 604.

In this embodiment, it is best that the second rotating disk 601 is provided with a regular hexagonal convex portion, and the blood sample tray 602 is provided with a regular twenty-four-sided concave portion.

Specifically, the blood sample tray 602 includes a tray body 605 and a plurality of carriers 606 mounted on the tray body 605, wherein the regular polygonal convex portion 603 or the regular polygonal concave portion 604 is installed on the tray body 605, and the carrier 606 is used to place blood sample testing tubes containing blood samples. The plurality of carriers 606 are distributed in a circle around the tray body 605 as the center and are enclosed in at least one circle. In specific implementation, it is best that a plurality of carriers 606 are enclosed in at least two circles, and the carriers 606 located in the inner circle and the carriers 606 located in the outer circle are misaligned, so as to obtain the barcode of the blood sample testing tubes on the carrier 606 in the inner circle.

Furthermore, the carrier 606 is provided with a second opening 607, when implemented, the blood sample testing tube is placed on the carrier 606 so that the barcode on the blood sample testing tube is located at the second opening 607 to facilitate to obtain the barcode on the blood sample testing tube.

The carrier 606 includes a round tube-shaped fixing seat 608 and a blood sample testing tube-loading seat 609 inserted on the fixing seat 608, the fixing seat 608 is installed on the tray body 605, and the second opening 607 is provided on the peripheral side of the blood sample testing tube-loading seat 609. In this embodiment, the carrier 606 adopts a split-type design to facilitate to install the blood sample testing tube, of course, the carrier 606 can also adopt an unit-type design.

Further, the tray body 605 is provided with a containing groove 617 for containing the fixing seat 608, when implemented, a plurality of fixing seats 608 are distributed in a circle around the tray body 605 as the center and are enclosed in at least one circle.

The blood sample-loading device 600 also includes a transmission mechanism 610 that drives the second rotating disk 601 to rotate, the transmission mechanism 610 includes a transmission shaft 611, a second driving wheel 612, a second driven wheel 613, and a motor 614, wherein one end of the transmission shaft 611 is equipped with the second rotating disk 601, the opposite end is equipped with the second driven wheel 612, and the second driving wheel 612 is connected to the rotating shaft of the motor 614 and meshes with the second driven wheel 612. During implementation, the motor 614 drives the second driving wheel 612 to rotate, the second driving wheel 612 then drives the second driven wheel 612 to rotate, and the second driven wheel 612 drives the second rotating disk 601 to rotate through the transmission shaft 611.

Further, the blood sample-loading device further includes a barcode reader 615, which is arranged close to the blood sample tray 602, and is used to read the barcode on the blood sample testing tube. When implemented, the light emitted by the barcode reader 615 is a plane light 616, as the carriers 606 at the outer circle and the carriers 606 at the inner circle are misaligned, the plane light 616 emitted by the barcode reader 615 can pass through the gap between the carriers 606 at the outer circle to enter the inner circle, so that the barcode reader 615 can read the barcode of the blood sample testing tube on the carriers 606 at the inner circle.

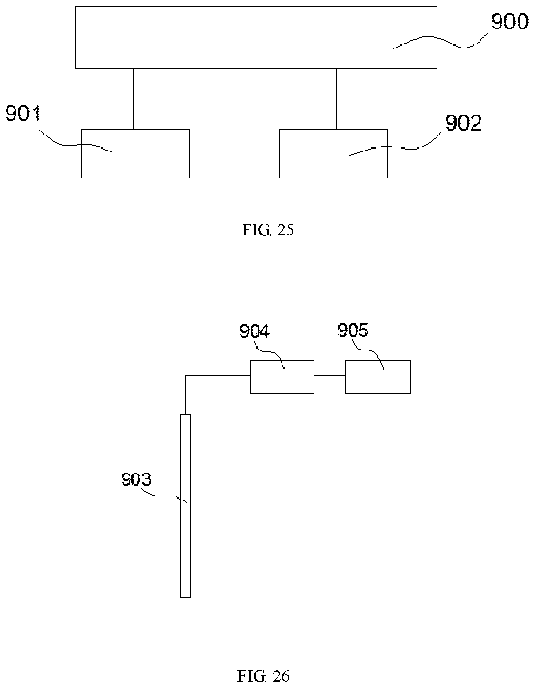

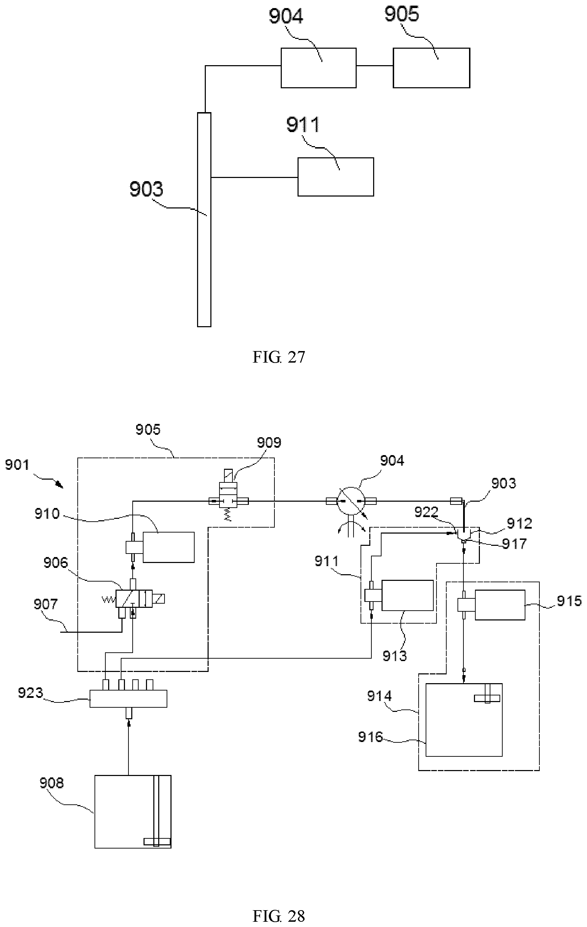

The liquid adding device is used to automatically add reagents and blood samples to the testing cup, in this embodiment, as shown in FIGs.25-29, it specifically includes a fluid path system 900 and a liquid-taking device 930 connected to the fluid path system 900, wherein the fluid path system 900 includes an independent blood sample fluid path system 901 and an independent reagent fluid path system 902, the blood sample fluid path system 901 is used for automatically adding blood during detecting blood coagulation data, the reagent fluid path system 902 is used for automatically adding reagents during detecting blood coagulation data.

Specifically, in this embodiment, the blood sample fluid path system 901 includes a blood-suctioning member 903, a first suction-controlling member 904, and a suctioning member-cleaning device 905, wherein the blood-suctioning member 903 is used for suctioning blood, it has a hollow syringe needle-like shape as a whole, when suctioning blood, its lower end goes deep into the blood sample testing tube.

The first suction-controlling member 904 leads to the blood-suctioning member 903, specifically connected to the upper end of the blood-suctioning member 903 through a hose, when implemented, the first suction-controlling member 904 can be a plunger pump. The working principle is that when the first suction-controlling member 904 operates, it controls the blood-suctioning member 903 to form negative pressure, and to suction the blood from a blood sample testing tube, when the first suction-controlling member 904 stops, it controls the blood-suctioning member 903 to release the suctioned blood into a testing cup 800.

The suctioning member-cleaning device 905 is connected to the blood-suctioning member 903, used for thoroughly cleaning the inner wall of the blood-suctioning member 903 after the blood-suctioning member 903 releases blood into the testing cup. Specifically, in this embodiment, the suctioning member-cleaning device 905 includes a first control valve 906, wherein the first control valve 906 leads to the blood-suctioning member 903 through the first suction-controlling member 904 , and the first control valve 906 further leads to a gas source and a water source. When implemented, the first control valve 906 can be a control valve with at least three joint openings, such as a two-position three-way valve, and of course, it can also be a four-way valve, a five-way valve, and the like. One of the joint openings of the three-way valve leads to the first suction-controlling member 904, and the other two joint openings are connected to the air source 907 and the water source 908, respectively. When implemented, the water source 908 can be clean water, and the clean water is stored in a water tank, that is, the three-way valve is connected to the water tank, the air source 907 can be air. In addition, the first control valve 906 and the water source 908 can also be connected with each other through a connector 923.

When cleaning, firstly the three-way control valve 906 controls the joint opening connected to the water tank 908 to be in an on state, and the joint opening connected to the air source 907 to be in an off state, after that, the first suction-controlling member 904 controls it to make the water source suctioned from water tank 908 entry the blood-suctioning member 903 through each connecting hose, so as to wash the inner wall of the blood-suctioning member 903, after washing, in order to blow off the cleaning liquid remaining on the inner wall of the blood-suctioning member 903, the three-way control valve 906 controls the joint opening connected to the water tank 908 to be in an off state, and the joint opening connected to the air source 907 to be in an on state, after that, the first suction-controlling member 904 controls it to make the air source 907 suctioned into the blood-suctioning member 903 through each connecting hose, so as to blow off the cleaning liquid remaining on the inner wall of the blood-suctioning member 903. In this way, the blood-suctioning member 903 is deeply cleaned and blown dry, so that it can suction other types of blood samples and avoid cross-contamination between different blood samples.

Preferably, in order to prevent blood from flowing into the water tank 908 or the air source 907 through the first suction-controlling member 904, or to avoid the backflow of the water source 908 and the air source 907, a check valve 909 can be addedly provided between the first control valve 906 and the first suction-controlling member 904, that is, restricts the water source 908 and the air source 907 to unidirectional flow into the blood-suctioning member 903, if reversed flow, the passage is disconnected. The check valve 909 is closed when the first suction-controlling member 904 is started to control the blood-suctioning member 904 to suction blood, and is opened when the suctioning member-cleaning device 23 is operating.

More preferably, a first diaphragm pump 910 is connected between the check valve 909 and the first control valve 906, used for matching with the first suction-controlling member 904 to achieve suction control to the water source 908 and the air source 907.

Further, in order to clean the outer wall of the blood-suctioning member while cleaning the inner wall of the blood-suctioning member, the blood sample fluid path system 901 of the embodiment according to the invention further includes a suctioning-member outer-wall-cleaning device 911, which specifically includes a suctioning member-cleaning trough 912 and a second diaphragm pump 913, wherein the blood-suctioning member 903 extends into the suctioning member-cleaning trough 912, and the suctioning member-cleaning trough 912 is connected to the above-mentioned water source (ie, the water tank 4) through the second diaphragm pump 913, that is, the second diaphragm pump 913 is connected with the suctioning member-cleaning trough 912 and the water tank 908, used for controlling the water in the water tank 908 to be suctioned into the suctioning member-cleaning trough 912, so as to clean the outer wall of the blood-suctioning member 903 inside the suctioning member-cleaning trough 912.

Specifically, a water source inlet 922 is provided on the side wall of the suctioning member-cleaning trough 912 close to its top, and the water source inlet 922 leads to the second diaphragm pump 913. Preferably, in order to ensure that the blood contaminated on the outer wall of the blood-suctioning member 903 can be cleaned when the blood is suctioned, in the embodiment according to the invention, the height of the water source inlet 922 in the suctioning member-cleaning trough 912 is set to be higher than the height of the blood sample on the outer wall of the blood-suctioning member 903 extending into the suctioning member-cleaning trough 912, that is, above the blood sample on the outer wall of the blood-suctioning member 903, so as to ensure that the water entering the blood-suctioning member 903 can completely covers and washes the blood sample on the outer wall of the blood-suctioning member 903 to make the blood sample on the outer wall of the blood-suctioning member 903 cleaned thoroughly.

Further, the blood sample fluid path system in the embodiment according to the invention may further include a sewage-collecting device 914, which is connected to the suctioning member-cleaning trough 912, and is used to collect and discharge the sewage generated after cleaning the suctioning member-cleaning device 905 and the suctioning-member outer-wall-cleaning device 911. Specifically, in this embodiment, the sewage-collecting device 914 includes a third diaphragm pump 915 and a sewage barrel 916 connected to the third diaphragm pump 915, wherein a sewage outlet 917 is provided on the suctioning member-cleaning trough 912, specifically is arranged at the bottom. The third diaphragm pump 915 is connected with the sewage outlet 917 and the sewage barrel 916, and is used to control the sewage generated in the suctioning member-cleaning trough 912 after cleaning to be collected and discharged into the sewage barrel 916.

In this embodiment, the reagent fluid path system 902 specifically includes at least one reagent-suctioning member 918 and a second suction-controlling member 919 connected to the reagent-suctioning member 918, wherein the reagent-suctioning member 918 is used to suction the reagents required for the test, when implemented, the reagent-suctioning member 918 is generally set to be several ones, each of which suctions one kind of reagents, in this embodiment, seven reagent-suctioning members 918 are provided, that is, suction 7 kinds of reagents correspondingly, of course, the number of reagent-suctioning members 918 is not limited and can be set according to the requirements for the reagents. Each reagent-suctioning member 918 also has a hollow syringe needle-like shape as a whole, when suctioning reagents, its lower end goes deep into the reagent testing tube (not shown in the figures). In addition, 7 reagent-suctioning members 918 are all connected to an integrated rod 920, and a connection opening 921 is provided on the integrated rod 920 at a position corresponding to each reagent-suctioning member 918.

The second suction-controlling member 919 is selectively connected to the connection opening 921 on the integrated rod 920 as required to realize the connection with the reagent-suctioning member 918 corresponding to the connection opening 921. Since each reagent-suctioning member 918 suctions one kind of reagents correspondingly, there is no cross-contamination between each other, so the reagent fluid path system here may not be provided with a corresponding cleaning device.

The liquid-taking device 930 realizes automatic driving during adding blood samples and reagents through the cooperation of three designed driving mechanisms (a blood suction-driving mechanism 931, a reagent suction-driving mechanism 932 and a rotationally-driving mechanism 933).

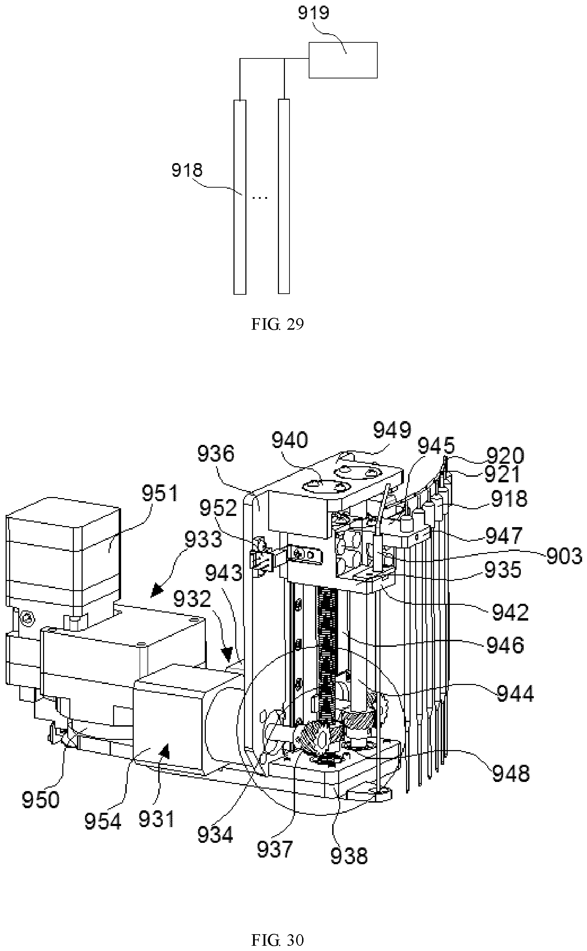

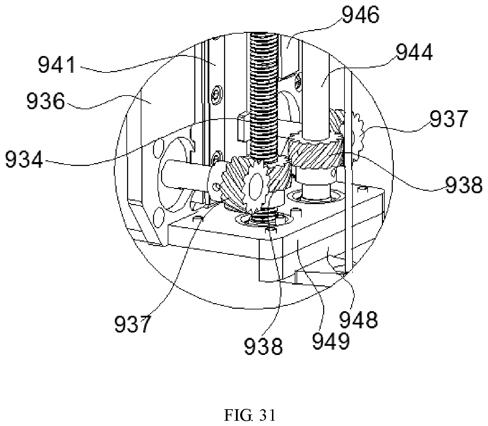

As shown in FIGs.30-31, the liquid-taking device 930 disclosed in the embodiments of the invention includes a blood suction-driving mechanism 931, a reagent suction-driving mechanism 932, and a rotationally-driving mechanism 933, wherein the blood suction-driving mechanism 931 is connected to a blood-suctioning member 903, and the blood-suctioning member 903 is driven to extend into or out of a blood sample testing tube, the reagent suction-driving mechanism 932 is connected to a reagent-suctioning member 918, the reagent-suctioning member 918 is driven to extend into or out of a reagent testing tube (not shown in the figures), the rotationally-driving mechanism 933 is connected to both the blood suction-driving mechanism 931 and the reagent suction-driving mechanism 932, used to match with both the mechanisms 931, 932 to drive both to rotate in the first direction, and move to the corresponding blood sample-loading device 600 and the reagent-storing device 500, respectively. The first direction in this embodiment is the horizontal direction.

Specifically, the blood suction-driving mechanism 931 includes a sixth driving source, a first gear assembly, a first screw rod 934 and a first sliding stand 935, wherein the sixth driving source is fixed on a first mounting plate 936 vertically arranged, specifically fixed to the lower end of the first mounting plate 936, the sixth driving source can be a first motor, when implemented, and the driving shaft of the first motor horizontally passes through the first mounting plate 936.

The first gear assembly includes a first gear 937 and a second gear 938, wherein the first gear 937 is fixed to the top end of the driving shaft of the first motor (that is, the end that passes through the first mounting plate 936 ), driven to rotate axially by the driving shaft of the first motor, the second gear 938 encircles the first screw rod 934 and meshes perpendicularly with the first gear 937, that is, the second gear 938 rotates in a radial direction perpendicular to the axial direction while the first gear 937 is rotating, that is, achieves a 90° turn.

The first screw rod 934 is vertically arranged, that is, parallel or approximately parallel to the plane where the first mounting plate 936 is located, in this embodiment, it is threadedly connected to the second gear 938 and can be driven with the rotation of the second gear 938 to rotate in a radial direction contrary to the rotation direction with respect to the second gear 938. Specifically, in this embodiment, the upper and lower ends of the first screw rod 934 are rotatably connected to a second mounting plate 939, and the second mounting plate 939 and the first mounting plate 936 can be integrally formed or arranged separately. When implemented, the rotational connection between both the ends of the first screw rod 934 and the second mounting plate 939 can be achieved through a rotary bearing 940.

The first sliding stand 935 is slidably connected to the first screw rod 934, and moves up and down along with the rotation of the first screw rod 934. Specifically, in this embodiment, the first sliding stand 935 is located between both the second mounting plates 939 and is arranged at the upper end of the first screw rod 934. Preferably, the first sliding stand 935 is also guided by a first sliding rail 941 to move in the vertical direction, and a first sliding groove matching with the first sliding rail 941 is arranged on the inner surface of the first sliding stand 935 abutting onto the first sliding rail 941 (not shown in the figures). In this embodiment, the first sliding rail 941 is vertically arranged on the first mounting plate 936.

The outer surface of the first sliding stand 935 (the side opposite to the first sliding groove) protrudes outwards to form a first fixing bump 942, and the blood-suctioning member 903 vertically passes through the first fixing bump 942 and is fixed inside the first fixed bump 942. The first sliding stand 935 is driven by the first screw rod 934 to make the blood-suctioning member 903 extend into or out of the blood sample testing tube. In this embodiment, the first sliding stand 935 is connected to one blood-suctioning member 903, and the blood-suctioning member 903 is used to suction the blood sample of different blood groups.

The reagent suction-driving mechanism 932 has a structure similar to the blood suction-driving mechanism 931, in this embodiment, specifically includes a seventh driving source 943, a second gear assembly, a second screw rod 944 and a second sliding stand 945, wherein the seventh driving source 943 is also fixed on the first mounting plate 936, specifically fixed to the lower end of the first mounting plate 936, arranged alongside the sixth driving source 934, the seventh driving source 943 can be a motor, when implemented, and the driving shaft of the motor 943 horizontally passes through the first mounting plate 936.

With the same structure as the first gear assembly, the second gear assembly includes a first gear 937 and a second gear 938, wherein the first gear 937 is fixed to the top end of the driving shaft of the second motor 943 (that is, the end that passes through the first mounting plate 936 ), driven to rotate axially by the driving shaft of the second motor 943, the second gear 938 encircles the second screw rod 944 and meshes perpendicularly with the first gear 937, that is, the second gear 938 rotates in a radial direction perpendicular to the axial direction while the first gear 937 rotates, that is, achieves a 90° turn. Of course, the invention is not limited to the gear assembly structure here, and other transmission structures that can realize the transmission between the driving source and the screw rod are applicable to the invention.

The second screw rod 944 is vertically arranged, that is, parallel or approximately parallel to the plane where the first mounting plate 936 is located, in this embodiment, it is threadedly connected to the second gear 938 and can be driven with the rotation of the second gear 938 to rotate in a radial direction contrary to the rotation direction with respect to the second gear 938. Specifically, in this embodiment, the upper and lower ends of the second screw rod 944 are also rotatably connected to the second mounting plate 939, respectively. When implemented, the rotational connection between both the ends of the second screw rod 944 and the second mounting plate 939 can be achieved through the rotary bearing 940 in the same way.

The second sliding stand 945 is slidably connected to the second screw rod 944, and moves up and down along with the rotation of the second screw rod 944. Specifically, in this embodiment, the second sliding stand 945 is located between both the second mounting plates 939 and is arranged at the upper end of the second screw rod 944. Preferably, the second sliding stand 945 is also guided by a second sliding rail 946 to move in the vertical direction, and a second sliding groove matching with the second sliding rail 946 is arranged on the inner surface of the second sliding stand 945 abutting onto the second sliding rail 946 (not shown in the figures). In this embodiment, the second sliding rail 946 is vertically arranged on the first mounting plate 936.

The outer surface of the second sliding stand 945 (the side opposite to the second sliding groove) protrudes outwards to form a second fixing bump 947, and the reagent-suctioning member 918 vertically passes through the second fixing bump 947 and is fixed inside the second fixed bump 947. The second sliding stand 945 is driven by the second screw rod 944 to make the reagent-suctioning member 918 extend into or out of the reagent testing tube. In this embodiment, the second sliding stand 945 is connected to a plurality of reagent-suctioning members 918, preferably seven, and each reagent-suctioning member 918 suctions one kind of reagents correspondingly. In implementation, a plurality of reagent-suctioning members 918 can all be connected to an integrated rod 920, and a connection opening 921 is provided on the integrated rod 920 at a position corresponding to each reagent-suctioning member 918.

The first motor, the second motor 943, the first mounting plate 936 and the second mounting plate 939 are all integrated and fixed on a base 948.

The rotationally-driving mechanism 933 is connected to the base 948, so as to realize the connection with both the blood suction-driving mechanism 931 and the reagent suction-driving mechanism 932, and is used to drive both the mechanisms 931, 932 to rotate in the horizontal direction, so that both the mechanisms 931, 932 move to the corresponding blood sample testing tube and reagent testing tube, respectively, which match with both the mechanisms 931, 932 to complete automatically driving the addition of the blood and reagents.

The rotationally-driving mechanism 933 specifically includes an eighth driving source 949 and a rotating shaft 950, when implemented, the eighth driving source 949 may be a third motor 951, which is connected to the rotating shaft 950 to drive the rotating shaft 950 to rotate. The rotating shaft 950 is connected to the base 948 described above, and the base 948 is driven to rotate in a horizontal direction, thereby synchronously driving the blood suction-driving mechanism 931 and the reagent suction-driving mechanism 932 to move in the horizontal direction.

Preferably, the first mounting plate 936 is also provided with a liquid level sensor 952 for detecting the liquid level inside the blood-suctioning member 903 and the reagent-suctioning member 918, so that the blood-suctioning member 903 and the reagent-suctioning member 918 suction blood samples and reagents to a preset liquid level, respectively.

Based on the automatic thrombo elastometer device, a test method of automatic thrombo elastometer comprises:
S1 fully loading testing cups on the testing cup-loading device,
S2 the test-driving device driving its cup-clamping mechanism to first move to the testing cup-loading device so as to remove a testing cup, and then move the clamped testing cup to the test passage so as to detach the cup lid, after that, transferring the testing cup from which the lid is detached to the liquid-adding device so as to add the reagent and the blood sample to the testing cup, respectively, after adding, moving the testing cup and loading it on the test passage,
S3 detecting blood coagulation data on the test passage,
S4 after completing detection, the test-driving device removing the testing cup from the test passage,
S5 after completing detection, the test-driving device driving the cup-clamping mechanism to unload the testing cup to the waste-collecting device,
S6 repeating the above steps S1-S5.

The specific implementation process of each step can refer to the description in the above-mentioned device, and will not be repeated here.

The technical content and technical features of the invention have been disclosed as above, but a person skilled in the art may still make various replacements and modifications based on the teachings and disclosures. Therefore, the protection scope of the invention should not be limited to the content disclosed in the embodiments, but should include various replacements and modifications that do not deviate from the invention, and are covered by the claims of this patent application.

## Claims

1. A full-automatic thrombo elastometer device, wherein said device comprises a control device (100), a testing cup-loading device (200) connected to said control device (100), a test-driving device (300), a plurality of test passages (400), a reagent-storing device (500), a blood sample-loading device (600), and a liquid-adding device,
wherein said testing cup-loading device (200) is configured to have loaded therein a plurality of testing cups (800) required by a test,
said test-driving device (300) includes a cup-clamping mechanism (301) and a test-driving mechanism connected to said cup-clamping mechanism (301), said test-driving mechanism is configured to drive said cup-clamping mechanism (301) to move to said testing cup-loading device (200), each test passage (400), and said liquid-adding device, respectively, so as to complete at least automatically taking a testing cup (800) at said testing cup-loading device (200), and cooperatively carrying out removing a cup lid (407) at each test passage (400), adding a reagent and a blood sample at said liquid-adding device, and testing blood at each test passage (400),
said test passage is used to automatically complete testing blood,
each test passage (400) includes a cup lid-beating device, a rotating mechanism (409) which is configured to connect to a testing cup (800), an up-down-driving mechanism (413) and a testing rod (405), said cup lid-beating device is configured to unload a cup lid (407) from said testing rod (405) into said testing cup (800), said rotating mechanism (409) is configured to control a testing cup (800) to rotate during a test, said up-down-driving mechanism (413) is configured to drive said testing cup (800) to move to a set test height,
said reagent-storing device (500) is configured to have loaded therein a plurality of reagent testing tubes, and each reagent testing tube is configured to contain a reagent,
said blood sample-loading device (600) is configured to have loaded therein a plurality of blood sample testing tubes, and each blood sample testing tube is configured to contain a blood sample,
said liquid-adding device is configured to move to said reagent-storing device (500) and said blood sample-loading device (600), respectively, and suction a reagent and a blood sample, respectively, and add the suctioned reagent and blood sample to a testing cup (800) for a test, respectively.

2. The full-automatic thrombo elastometer device according to claim 1, wherein said testing cup-loading device (200) includes a testing cup tray (201) and a first rotating disk (202), said testing cup tray (201) is installed on said first rotating disk (202), and is configured to have loaded therein said plurality of testing cups (800).

3. The full-automatic thrombo elastometer device according to claim 1, wherein said test-driving mechanism includes a first up-down-driving mechanism (302), a rotationally-driving mechanism (303) and a horizontally-driving mechanism (304) all connected to said cup-clamping mechanism (301), said first up-down-driving mechanism (302), said rotationally-driving mechanism (303) and said horizontally-driving mechanism (304) are configured to cooperatively drive said cup-clamping mechanism (301) so as to move to said testing cup-loading device (200), each test passage (400) and said liquid-adding device, respectively, and wherein the up-down-driving mechanism of each test passage is a second up-down-driving mechanism.

4. The full-automatic thrombo elastometer device according to claim 1, wherein
said liquid-adding device includes a fluid path system (900), and a blood suction-driving mechanism (931), a reagent suction-driving mechanism (932) and a rotationally-driving mechanism (933) that are connected to said fluid path system (900), said fluid path system (900) includes a blood-suctioning member (903) and a reagent-suctioning member (918), said blood suction-driving mechanism (931) is connected with said blood-suctioning member (903) to drive said blood-suctioning member (903) to extend into or out of one of said plurality of blood sample testing tubes,
said reagent suction-driving mechanism (932) is connected with said reagent-suctioning member (918) to drive said reagent-suctioning member (918) to extend into or out of one of said plurality of reagent testing tubes,
said rotationally-driving mechanism (933) is connected to both said blood suction-driving mechanism (931) and said reagent suction-driving mechanism (932), and is configured to drive both to move to said reagent-storing device (500) and said blood sample-loading device (600), respectively.

5. The full-automatic thrombo elastometer device according to claim 1, wherein said reagent-storing device (500) includes a housing (501), a transmitting base (502) installed inside said housing (501), a transmitting drawer (503) which is configured to have placed therein the reagents, and an interlocking mechanism, said interlocking mechanism is configured to realize the press-locking or press-unlocking between said transmitting drawer (503) and said transmitting base (502), said transmitting drawer (503) is movably installed on said transmitting base (502).

6. The full-automatic thrombo elastometer device according to claim 1, wherein said blood sample-loading device (600) includes a second rotating disk (601) and a blood sample tray (602) installed on said second rotating disk (601), said blood sample tray (602) is configured to have loaded therein a plurality of blood sample testing tubes.

7. The full-automatic thrombo elastometer device according to claim 1, wherein said thrombo elastometer device further includes a waste-collecting device (953), said waste-collecting device (953) is configured to collect the used testing cups (800) transferred by said test-driving device (300), said test-driving mechanism is configured to drive said cup-clamping mechanism (301) to unload the used testing cups (800) to the waste-collecting device (953).

8. The full-automatic thrombo elastometer device according to claim 7, wherein said thrombo elastometer device further includes an outer housing (700), said control device (100), said testing cup-loading device (200), said test-driving device (300), said plurality of said test passages (400), said reagent-storing device (500), said blood sample-loading device (600), said liquid-adding device and said waste-collecting device (953) are all integrated inside said outer housing (700).

9. The full-automatic thrombo elastometer device according to claim 5, wherein said interlocking mechanism includes a locking assembly (505) installed on said transmitting base (502) and a tracking groove (508) installed on said transmitting drawer (503), the press-locking or press-unlocking is realized between said transmitting drawer (503) and said transmitting base (502) by matching said locking assembly (505) with said tracking groove (508).

10. The full-automatic thrombo elastometer device according to claim 9, wherein said tracking groove (508) includes a first opening (510), a first guiding groove (511), a limiting groove (512) and a second guiding groove (513), one end of said limiting groove (512) leads to one end of said first guiding groove (511), the opposite end leads to one end of said second guiding groove (513), and the other ends of said first guiding groove (511) and said second guiding groove (513) both lead to said first opening (510).

11. A test method using said full-automatic thrombo elastometer device according to any one of claims 7-8, **characterized in that** said method comprises the following steps,
S1 fully loading testing cups (800) on the testing cup-loading device (200),
S2 the test-driving device (300) driving its cup-clamping mechanism (301) to first move to the testing cup-loading device (200) so as to remove said testing cup (800), and then move the clamped testing cup (800) to the test passage (400) so as to detach the cup lid (407), after that, transferring the testing cup (800) from which the lid (407) is detached to the liquid-adding device so as to add the reagent and the blood sample to the testing cup (800), respectively, after adding, moving the testing cup (800) and loading it on the test passage (400),
S3 detecting blood coagulation data on the test passage (400),
S4 after completing detection, the test-driving device (300) removing the testing cup (800) from the test passage (400),
S5 after completing detection, the test-driving device (300) driving the cup-clamping mechanism (301) to unload the testing cup (800) to the waste-collecting device (953),
S6 repeating the above steps S1-S5.

## Patentansprüche

1. Vollautomatische Thromboelastometervorrichtung, wobei die Thromboelastometervorrichtung eine Steuervorrichtung (100), eine mit der Steuervorrichtung (100) verbundene Testbecher-Ladevorrichtung (200), eine Testantriebsvorrichtung (300), eine Vielzahl von Testdurchgängen (400), eine Reagenzienspeichervorrichtung (500), eine Blutproben-Ladevorrichtung (600) und eine Flüssigkeitszugabevorrichtung umfasst,
wobei die Testbecher-Ladevorrichtung (200) so konfiguriert ist, dass sie eine Vielzahl von Testbechern (800) darin lädt, die für einen Test erforderlich sind,
wobei die Testantriebsvorrichtung (300) einen Becherklemmmechanismus (301) und einen Testantriebsmechanismus, der mit dem Becherklemmmechanismus (301) verbunden ist, umfasst, wobei der Testantriebsmechanismus so konfiguriert ist, dass er den Becherklemmmechanismus (301) antreibt, um sich jeweils zu der Testbecher-Ladevorrichtung (200), jedem Testdurchgang (400) und der Flüssigkeitszugabevorrichtung zu bewegen, um zumindest die automatische Entnahme eines Testbechers (800) an der Testbecher-Ladevorrichtung (200) und die kooperative Durchführung des Entfernens eines Becherdeckels (407) an jedem Testdurchgang (400), die Zugabe eines Reagenz und einer Blutprobe an der Flüssigkeitszugabevorrichtung und das Testen von Blut an jedem Testdurchgang (400) abzuschließen,
wobei der Testdurchgang der automatischen Vervollständigung des Bluttests dient,
wobei jeder Testdurchgang (400) eine Becherdeckel-Schlagvorrichtung, einen Drehmechanismus (409), der so konfiguriert ist, dass er mit einem Testbecher (800) verbunden ist, einen Auf-Ab-Antriebsmechanismus (413) und eine Teststange (405) umfasst, wobei die Becherdeckel-Schlagvorrichtung so konfiguriert ist, dass sie einen Becherdeckel (407) von der Teststange (405) in den Testbecher (800) entlädt, wobei der Drehmechanismus (409) so konfiguriert ist, dass er einen Testbecher (800) so steuert, dass er sich während eines Test dreht, wobei der Auf-Ab-Antriebsmechanismus (413) so konfiguriert ist, dass er den Testbecher (800) so antreibt, dass er sich auf eine eingestellte Testhöhe bewegt,
wobei die Reagenzienspeichervorrichtung (500) so konfiguriert ist, dass eine Vielzahl von Reagenzteströhrchen darin geladen ist, und wobei jedes Reagenzteströhrchen so konfiguriert ist, dass es ein Reagenz enthält,
wobei die Blutproben-Ladevorrichtung (600) so konfiguriert ist, dass darin eine Vielzahl von Blutproben-Teströhrchen geladen ist, und wobei jedes Blutproben-Teströhrchen so konfiguriert ist, dass es eine Blutprobe enthält,
wobei die Flüssigkeitszugabevorrichtung so konfiguriert ist, dass sie sich zu der Reagenzienspeichervorrichtung (500) bzw. der Blutproben-Ladevorrichtung (600) bewegt und ein Reagenz bzw. eine Blutprobe absaugt und das abgesaugte Reagenz und die Blutprobein einen Testbecher (800) für einen Test hinzufügt.

2. Vollautomatische Thromboelastometervorrichtung nach Anspruch 1, wobei die Testbecher-Ladevorrichtung (200) einen Testbecherteller (201) und eine erste rotierende Scheibe (202) umfasst, wobei der Testbecherteller (201)auf der ersten rotierenden Scheibe (202) installiert ist und so konfiguriert ist, dass darin die mehreren Testbecher (800) geladen werden können.

3. Vollautomatische Thromboelastometervorrichtung nach Anspruch 1, wobei der Testantriebsvorrichtung einen ersten Aufwärts/Abwärts-Antriebsmechanismus (302), einen Rotations-Antriebsmechanismus (303) und einen Horizontal-Antriebsmechanismus (304) umfasst, die alle mit dem Becherklemmmechanismus (301) verbunden sind, wobei der erste Aufwärts/Abwärts-Antriebsmechanismus (302), der Rotations-Antriebsmechanismus (303) und der Horizontal-Antriebsmechanismus (304) so konfiguriert sind, dass sie zusammenwirkend den Becherklemmmechanismus (301) so antreiben, dass sie sich zu der Testbecher-Ladevorrichtung (200), jedem Testdurchgang (400) bzw. der Flüssigkeitszugabevorrichtung bewegen, und wobei der Aufwärts/Abwärts-Antriebsmechanismus jedes Testdurchgangs ein zweiter Aufwärts/Abwärts-Antriebsmechanismus ist.

4. Vollautomatische Thromboelastometervorrichtung nach Anspruch 1, wobei die Flüssigkeitszugabevorrichtung ein Flüssigkeitspfadsystem (900) und einen Blutsaug-Antriebsmechanismus (931), einen Reagenzsaug-Antriebsmechanismus (932) und einen Rotations-Antriebsmechanismus (933) umfasst, die mit dem Flüssigkeitspfadsystem (900) verbunden sind, wobei das Flüssigkeitspfadsystem (900) ein Blutsaugelement (903) und ein Reagenzabsaugungselement (918) umfasst, wobei der Blutsaug-Antriebsmechanismus (931) mit dem Blutsaugelement (903) verbunden ist, um das Blutsaugelement (903) so anzutreiben, dass es sich in eines der Vielzahl von Blutprobenteströhrchen hinein oder aus diesem heraus erstreckt,
wobei der Reagenzsaug-Antriebsmechanismus (932) mit dem Reagenzsaugelement (918) verbunden ist, um das Reagenzsaugelement (918) anzutreiben, damit es sich in eines der Vielzahl von Reagenzteströhrchen hinein oder aus diesem heraus erstreckt, und wobei das Reagenzsaugelement (918) mit den Vielzahl von Reagenzteströhrchen verbunden ist.
wobei der Rotations-Antriebsmechanismus (933) sowohl mit dem Blutsaug-Antriebsmechanismus (931) als auch mit dem Reagenzansaug-Antriebsmechanismus (932) verbunden und so konfiguriert ist, dass er beide antreibt, um sich zu der Reagenzienspeichervorrichtung (500) bzw. der Blutproben-Ladevorrichtung (600) zu bewegen.

5. Vollautomatische Thromboelastometervorrichtung nach Anspruch 1, wobei die Reagenzienspeichervorrichtung (500) ein Gehäuse (501), eine Übertragungsbasis (502), die innerhalb des Gehäuses (501) installiert ist, eine Übertragungsschublade (503), die so konfiguriert ist, dass die Reagenzien darin untergebracht werden können, und einen Verriegelungsmechanismus umfasst, wobei der Verriegelungsmechanismus so konfiguriert ist, dass er die Druckverriegelung oder Druckentriegelung zwischen der Übertragungsschublade (503) und der Übertragungsbasis (502) realisiert, wobei die Übertragungsschublade (503) beweglich auf der Übertragungsbasis (502) installiert ist.

6. Vollautomatische Thromboelastometervorrichtung nach Anspruch 1, wobei die Blutproben-Ladevorrichtung (600) eine zweite rotierende Scheibe (601) und eine Blutprobenschale (602) umfasst, die auf der zweiten rotierenden Scheibe (601) installiert ist, wobei die Blutprobenschale (602) so konfiguriert ist, dass darin eine Vielzahl von Blutprobenteströhrchen geladen ist.

7. Vollautomatische Thromboelastometervorrichtung nach Anspruch 1, wobei die Thromboelastometervorrichtung ferner eine Abfallsammelvorrichtung (953) umfasst, wobei die Abfallsammelvorrichtung (953) so konfiguriert ist, dass sie die gebrauchten Testbecher (800), die von der Testantriebsvorrichtung (300) übertragen werden, sammelt, wobei der Testantriebsmechanismus so konfiguriert ist, dass er den Becherklemmmechanismus (301) antreibt, um die gebrauchten Testbecher (800) zu der Abfallsammelvorrichtung (953) zu entladen.

8. Vollautomatische Thromboelastometervorrichtung nach Anspruch 7, wobei die Thromboelastometervorrichtung ferner ein Außengehäuse (700), wobei die Steuervorrichtung (100), die Testbecher-Ladevorrichtung (200), die Testantriebsvorrichtung (300), die Vielzahl von Testdurchgänge (400), die Reagenzienspeichervorrichtung (500), die Blutproben-Ladevorrichtung (600), die Flüssigkeitszugabevorrichtung und die Abfallsammelvorrichtung (953) alle in das Außengehäuse (700) integriert sind.

9. Vollautomatische Thromboelastometervorrichtung nach Anspruch 5, wobei der Verriegelungsmechanismus eine Verriegelungsbaugruppe (505), die an der Übertragungsbasis (502) installiert ist, und eine Spurnut (508), die an der Übertragungsschublade (503) installiert ist, umfasst, wobei die Druckverriegelung oder Druckentriegelung zwischen der Übertragungsschublade (503) und der Übertragungsbasis (502) durch Zusammenpassen der Verriegelungsbaugruppe (505) mit der Spurnut (508) realisiert wird.

10. Vollautomatische Thromboelastometervorrichtung nach Anspruch 9, wobei die Spurnut (508) eine erste Öffnung (510), eine erste Führungsnut (511), eine Begrenzungsnut (512) und eine zweite Führungsnut (513) aufweist, wobei ein Ende der Begrenzungsnut (512) zu einem Ende der ersten Führungsnut (511) führt und das gegenüberliegende Ende zu einem Ende der zweiten Führungsnut (513) führt und ein Ende der zweiten Führungsnut (513), und wobei die anderen Enden der ersten Führungsnut (511) und der zweiten Führungsnut (513) beide zu der ersten Öffnung (510) führen.

11. Testverfahren unter Verwendung der Vollautomatischen Thromboelastometervorrichtung nach einem der Ansprüche 7 bis 8, **dadurch gekennzeichnet, dass** das Verfahren die folgenden Schritte umfasst:
S1: Vollständiges Laden der Testbecher (800) auf die Testbecher-Ladevorrichtung (200),
S2: Antreiben des Becherklemmmechanismus (301) durch die Testantriebsvorrichtung (300), um sich zuerst zur Testbecher-Ladevorrichtung (200) zu bewegen, um den Testbecher (800) zu entfernen, und dann Bewegen des eingeklemmten Testbecher (800) zum Testdurchgang (400), um den Becherdeckel (407) zu lösen, Überführen des Testbechers (800), von dem der Becherdeckel (407) abgenommen ist, zu der Flüssigkeitszugabevorrichtung, um das Reagenz bzw. die Blutprobe in den Testbecher (800) hinzuzufügen, und nach Zugabe, Bewegen des Testbechers (800) und Laden in den Testdurchgang (400),
S3: Erfassen von Blutgerinnungsdaten auf dem Testdurchgang (400),
S4: Nach Abschluss der Erfassung, Entfernen des Testbechers (800) durch die Testantriebsvorrichtung (300) aus dem Testdurchgang (400),
S5: Nach Abschluss der Erfassung, Antreiben des Becherklemmmechanismus (301) durch die Testantriebsvorrichtung (300), um den Testbecher (800) in die Abfallsammelvorrichtung (953) zu entladen, und
S6: Wiederholen der obigen Schritte S1 bis S5.

## Revendications

1. Dispositif de thromboélastométrie automatique, **caractérisé en ce que** ledit dispositif comprend un dispositif de commande (100), un dispositif de chargement de coupelle d'essai (200) relié audit dispositif de commande (100), un dispositif d'entraînement d'essai (300), plusieurs passages d'essai (400), un dispositif de stockage de réactif (500), un dispositif de chargement d'échantillon de sang (600), et un dispositif d'ajout de liquide,
dans lequel ledit dispositif de chargement de coupelle d'essai (200) est configuré pour contenir plusieurs coupelles d'essai (800) nécessaires à un essai,
ledit dispositif d'entraînement d'essai (300) comprend un mécanisme de serrage de coupelles (301) et un mécanisme d'entraînement d'essai connecté audit mécanisme de serrage de coupelles (301), ledit mécanisme d'entraînement d'essai étant configuré pour entraîner ledit mécanisme de serrage de coupelles (301) afin de le déplacer vers ledit dispositif de chargement de coupelle d'essai (200), chaque passage d'essai (400) et ledit dispositif d'ajout de liquide, respectivement, de manière à compléter au moins automatiquement la prise d'une coupelle d'essai (800) dans ledit dispositif de chargement de coupelle d'essai (200), et à effectuer ensemble le retrait d'un couvercle de coupelle (407) dans chaque passage d'essai (400), l'ajout d'un réactif et d'un échantillon de sang dans ledit dispositif d'ajout de liquide, et l'essai de sang dans chaque passage d'essai (400),
ce passage d'essai est utilisé pour compléter automatiquement l'analyse du sang,
chaque passage d'essai (400) comprend un dispositif pour couvercle rabattable de coupelle, un mécanisme en rotation (409) configuré pour se connecter à une coupelle d'essai (800), un mécanisme d'entraînement de haut en bas (413) et une tige d'essai (405), ledit dispositif pour couvercle rabattable de coupelle est configuré pour décharger un couvercle de coupelle (407) de ladite tige d'essai (405) dans ladite coupelle d'essai (800), ledit mécanisme de rotation (409) est configuré pour commander la rotation d'une coupelle d'essai (800) pendant un essai, ledit mécanisme d'entraînement de haut en bas (413) est configuré pour entraîner ladite coupelle d'essai (800) jusqu'à une hauteur d'essai définie,
ledit dispositif de stockage de réactif (500) est configuré pour contenir plusieurs tubes à essai de réactif, et chaque tube à essai de réactif est configuré pour contenir un réactif,
ledit dispositif de chargement d'échantillon de sang (600) est configuré pour contenir plusieurs tubes à essai d'échantillon de sang, et chaque tube à essai d'échantillon de sang est configuré pour contenir un échantillon de sang,
ledit dispositif d'ajout de liquide est configuré pour se déplacer vers ledit dispositif de stockage de réactif (500) et ledit dispositif de chargement d'échantillon de sang (600), respectivement, et pour aspirer un réactif et un échantillon de sang, respectivement, et ajouter le réactif et l'échantillon de sang aspirés à une coupelle d'essai (800) pour un essai, respectivement.

2. Dispositif de thromboélastométrie automatique selon la revendication 1, **caractérisé en ce que** ledit dispositif de chargement de coupelle d'essai (200) comprend un plateau pour coupelle d'essai (201) et un premier disque en rotation (202), ledit plateau pour coupelle d'essai (201) est installé sur ledit premier disque en rotation (202), et est configuré pour recevoir ladite pluralité de coupelles d'essai (800).

3. Dispositif de thromboélastométrie automatique selon la revendication 1, **caractérisé en ce que** ledit mécanisme d'entraînement d'essai comprend un premier mécanisme d'entraînement de haut en bas (302), un mécanisme d'entraînement en rotation (303) et un mécanisme d'entraînement horizontal (304), tous connectés audit mécanisme de serrage de coupelles (301), ledit premier mécanisme d'entraînement de haut en bas (302), ledit mécanisme d'entraînement en rotation (303) et ledit mécanisme d'entraînement horizontal (304) sont configurés pour entraîner en coopération ledit mécanisme de serrage de coupelles (301) de manière à se déplacer vers ledit dispositif de chargement de coupelles d'essai (200), chaque passage d'essai (400) et ledit dispositif d'ajout de liquide, respectivement, et dans lequel le mécanisme d'entraînement vers le bas de chaque passage d'essai est un second mécanisme d'entraînement vers le haut.

4. Dispositif de thromboélastométrie automatique selon la revendication 1, **caractérisé en ce que** ledit dispositif d'ajout de liquide comprend un système de circulation des fluides (900), et un mécanisme d'aspiration de sang (931), un mécanisme d'aspiration de réactif (932) et un mécanisme d'entraînement en rotation (933) qui sont connectés audit système de circulation des fluides (900), ledit système de circulation des fluides (900) comprend un élément d'aspiration de sang (903) et un élément d'aspiration de réactif (918), ledit mécanisme d'aspiration de sang (931) est relié audit élément d'aspiration de sang (903) pour entraîner ledit élément d'aspiration de sang (903) afin qu'il s'étende dans ou hors de l'un des tubes à essai d'échantillon de sang,
ledit mécanisme d'aspiration de réactif (932) est relié à l'élément d'aspiration de réactif (918) pour entraîner ledit élément d'aspiration de réactif (918) afin qu'il s'étende dans ou hors de l'un des tubes à essai de réactif,
ledit mécanisme d'entraînement en rotation (933) est relié à la fois audit mécanisme d'entraînement de l'aspiration de sang (931) et audit mécanisme d'entraînement de l'aspiration de réactif (932), et est configuré pour les entraîner tous deux à se déplacer vers ledit dispositif de stockage de réactif (500) et ledit dispositif de chargement de l'échantillon de sang (600), respectivement.

5. Dispositif de thromboélastométrie automatique selon la revendication 1, **caractérisé en ce que** ledit dispositif de stockage de réactif (500) comprend un boîtier (501), une base de transmission (502) installée à l'intérieur dudit boîtier (501), un tiroir de transmission (503) qui est configuré pour y placer les réactifs, et un mécanisme de verrouillage, ledit mécanisme de verrouillage étant configuré pour réaliser le verrouillage ou le déverrouillage par pression entre ledit tiroir de transmission (503) et ladite base de transmission (502), ledit tiroir de transmission (503) étant installé de manière mobile sur ladite base de transmission (502).

6. Dispositif de thromboélastométrie automatique selon la revendication 1, **caractérisé en ce que** ledit dispositif de chargement d'échantillon de sang (600) comprend un second disque en rotation (601) et un plateau d'échantillons de sang (602) installé sur ledit second disque en rotation (601), ledit plateau d'échantillons de sang (602) étant configuré pour contenir plusieurs tubes à essai d'échantillons de sang.

7. Dispositif de thromboélastométrie automatique selon la revendication 1, **caractérisé en ce que** ledit dispositif de thromboélastométrie comprend en outre un dispositif de collecte de déchets (953), ledit dispositif de collecte de déchets (953) est configuré pour collecter les coupelles d'essai usagées (800) transférées par ledit dispositif d'entraînement d'essais (300), ledit mécanisme d'entraînement d'essais est configuré pour entraîner ledit mécanisme de serrage de coupelles (301) afin de décharger les coupelles d'essai usagées (800) vers le dispositif de collecte de déchets (953).

8. Dispositif de thromboélastométrie automatique selon la revendication 7, **caractérisé en ce que** ledit dispositif de thromboélastométrie comprend en outre un boîtier extérieur (700), ledit dispositif de commande (100), ledit dispositif de chargement de coupelles d'essai (200), ledit dispositif d'entraînement d'essai (300), ladite pluralité de passages d'essai (400), ledit dispositif de stockage de réactif (500), ledit dispositif de chargement d'échantillon de sang (600), ledit dispositif d'ajout de liquide et ledit dispositif de collecte de déchets (953) sont tous intégrés dans ledit boîtier extérieur (700).

9. Dispositif de thromboélastométrie automatique selon la revendication 5, **caractérisé en ce que** ledit mécanisme de verrouillage comprend un ensemble de verrouillage (505) installé sur ladite base de transmission (502) et une rainure de guidage (508) installée sur ledit tiroir de transmission (503), le verrouillage ou le déverrouillage par pression est réalisé entre ledit tiroir de transmission (503) et ladite base de transmission (502) en faisant correspondre ledit ensemble de verrouillage (505) avec ladite rainure de guidage (508).

10. Dispositif de thromboélastométrie automatique selon la revendication 9, **caractérisé en ce que** ladite rainure de guidage (508) comprend une première ouverture (510), une première rainure de guidage (511), une rainure de limitation (512) et une seconde rainure de guidage (513), une extrémité de ladite rainure de limitation (512) mène à une extrémité de ladite première rainure de guidage (511), l'extrémité opposée mène à une extrémité de ladite deuxième rainure de guidage (513), et les autres extrémités de ladite première rainure de guidage (511) et de ladite deuxième rainure de guidage (513) mènent toutes deux à ladite première ouverture (510).

11. Méthode d'essai utilisant le dispositif de thromboélastométrie automatique selon l'une des revendications 7 à 8, **caractérisée en ce que** cette méthode comprend les étapes suivantes,
S1 chargement complet des coupelles d'essai (800) sur le dispositif de chargement de la coupelle d'essai (200),
S2 le dispositif d'entraînement d'essai (300) entraîne son mécanisme de serrage de coupelles (301) à se déplacer d'abord vers le dispositif de chargement de la coupelle d'essai (200) afin de retirer ladite coupelle d'essai (800), puis à déplacer la coupelle d'essai serrée (800) vers le passage d'essai (400) afin de détacher le couvercle de la coupelle (407), ensuite, transférer la coupelle d'essai (800) dont le couvercle (407) est détaché vers le dispositif d'ajout de liquide afin d'ajouter le réactif et l'échantillon de sang à la coupelle d'essai (800), respectivement, après l'ajout, déplacer la coupelle d'essai (800) et la charger sur le passage d'essai (400),
S3 détecter les données de coagulation du sang sur le passage d'essai (400),
S4 après avoir terminé la détection, le dispositif d'entraînement d'essai (300) retire la coupelle d'essai (800) du passage d'essai (400),
S5 une fois la détection terminée, le dispositif d'essai (300) actionne le mécanisme de serrage de coupelles (301) pour décharger la coupelle d'essai (800) dans le dispositif de collecte de déchets (953),
S6 répéter les étapes S1-S5 ci-dessus.
